# EUROPÄISCHE PATENTSCHRIFT

(11) **EP 1 515 965 B1**
(45) Veröffentlichungstag und Bekanntmachung des Hinweises auf die Patenterteilung: **06.10.2010**
(21) Anmeldenummer: 03759908.1
(22) Anmeldetag: 04.06.2003
(51) Int. Cl.: C07D 401/14, C07D 401/12, C07D 405/14, C07D 487/04, C07D 498/04, C07D 471/04, C07D 498/10, C07D 491/10, A61K 31/506, A61P 9/00

(54) **PHENYLAMINOPYRIMIDINE UND IHRE VERWENDUNG ALS RHO-KINASE INHIBITOREN**
PHENYLAMINOPYRIMIDINES AND THEIR USE AS RHO-KINASE INHIBITORS
PHENYLAMINOPYRIMIDINES ET LEUR UTILISATION EN TANT QU'INHIBITEURS DE LA RHO-KINASE

(30) Priorität: 17.06.2002 DE 10226943
(43) Veröffentlichungstag der Anmeldung: 23.03.2005
(73) Patentinhaber: Bayer Schering Pharma Aktiengesellschaft, 13353 Berlin (DE)
(72) Erfinder: FEURER, Achim, 69259 Wilhelmsfeld (DE); BENNABI, Samir, 42103 Wuppertal (DE); HECKROTH, Heike, 42327 Wuppertal (DE); ERGÜDEN, Jens, 42489 Wülfrath (DE); SCHENKE, Thomas, 51469 Bergisch Gladbach (DE); BAUSER, Marcus, 42115 Wuppertal (DE); KAST, Raimund, 42349 Wuppertal (DE); STASCH, Johannes-Peter, 42651 Solingen (DE); STAHL, Elke, 51467 Bergisch Gladbach (DE); MÜNTER, Klaus, 42489 Wülfrath (DE); LANG, Dieter, 42553 Velbert (DE); EHMKE, Heimo, 22301 Hamburg (DE)
(86) Internationale Anmeldenummer: PCT/EP2003/005827
(87) Internationale Veröffentlichungsnummer: WO 2003/106450

(56) Entgegenhaltungen:
- WO-A-01/28561
- WO-A-03/062225
- WO-A-03/062227
- US-A- 3 432 493
- US-A- 3 478 030

## Beschreibung

Die Erfindung betrifft Phenylaminopyrimidine, ein Verfahren zu ihrer Herstellung sowie ihre Verwendung zur Herstellung von Arzneimitteln zur Behandlung und/oder Prophylaxe von Krankheiten bei Menschen und Tieren, insbesondere von kardiovaskulären Erkrankungen.

Ein Anstieg der intrazellulären Calcium-Konzentration ist ein Hauptauslöser für die Kontraktion der Gefäßmuskulatur (Somlyo, A.P. und Himpens, B., FASEB J. 1989, 3, 2266-2276). Dies geschieht in erster Linie durch Agonisten wie z.B. Phenylephrin oder Thromboxan A2, die nach Stimulierung der Phosphatidylinositolkashade die Freisetzung von Calcium aus dem sarkoplasmatischen Retikulum bewirken. Die Erhöhung des intrazellulären Calciums aktiviert die MLC-Kinase (Myosin-Leichte-Ketten-Kinase), die die MLC-Untereinheiten des Myosinmoleküls phosphoryliert (Kamm, K.H. und Stull, J.T., Annu. Rev. Pharmacol.Toxicol. 1985, 25, 593-603). MLC-Phosphorylierung induziert die Glattmuskelkontraktion, MLC-Dephosphorylierung nach einer Reduktion der intrazellulären Calciumkonzentration resultiert in der Relaxation des Gefäße.

Neben der Calcium-abhängigen MLC-Phosphorylierung existiert noch ein weiterer zentraler aber Calcium-unabhängiger Regulationsmechanismus des Gefäßtonus. Hierbei handelt es sich um den Rho/Rho-Kinase-Signalweg (Noda, M. et al., FEBS Lett. 1995, 367, 246-250; Uehata, M. et al., Nature 1997, 389, 990-994; Fukata, Y. et al., Trends in Pharmacological Sciences 2001, 22, 32-39). Binden Agonisten wie z.B. Phenylephrin oder Thromboxan A2 an ihre Rezeptoren, so führt dies zur Aktivierung der kleinen G-Proteine Rho, die dann mit der Rho-Kinase interagieren und diese aktivieren. Die aktiviert Rho-Kinase inhibiert die Myosin-Phosphatase, nachdem sie eine Untereinheit des Enzyms phosphoryliert hat. Gleichzeitig phosphoryliert Rho-Kinase MLC an der Stelle, die auch von der MLC-Kinase phosphoryliert wird. Eine Hemmung der Myosin-Phosphatase sowie der Phosphorylierung von MLC induziert die Kontraktion der Gefäßmuskulatur. Im Gegensatz dazu führt eine Hemmung der Rho-Kinase zu einer Gefäßrelaxation. Inhibitoren der Rho-Kinase bewirken daher eine Senkung des Blutdruckes und eine Steigerung des koronaren Blutflusses.

Strukturell ähnliche Verbindungen sind in anderen Indikationen bzw. für andere Wirkmechanismen bekannt. So beschreiben beispielsweise US 3 478 030 und US 3 432 493 substituierte Aminopyrimidine, die den koronaren Blutfluss steigern können, dabei aber als Carboanhydrase-Inhibitoren wirken (J. Chem. Inf. Comp. Sciences 2002, 42, 94-102). Andere Pyrimidin-Derivate sind als Anti-Krebs- und Anti-HIV-Mittel (Debi, M.; Indian J. Exp. Biol. 1997, 35, 1205-1213) oder als cdk2-Inhibitoren (WO-A 01/64654) beschrieben.

Aufgabe der vorliegenden Erfindung ist die Bereitstellung von Arzneimitteln für die Behandlung von Erkrankungen, insbesondere von kardiovaskulären Erkrankungen.

Diese Aufgabe wird durch die Verbindungen der Formel (I) gelöst, die als Rho-Kinase-Inhibitoren wirken.

Gegenstand der vorliegenden Erfindung sind Verbindungen der Formel (I) 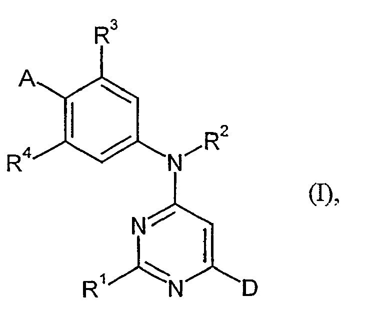 worin
- R¹: Amino oder Hydroxy bedeutet,
- R²: Wasserstoff, (C₁-C₆)-Alkyl oder (C₃-C₈)-Cycloalkyl bedeutet,
- R³ und R⁴: unabhängig voneinander Cyano, Wasserstoff, Fluor oder Chlor bedeuten,
- A: einen Rest 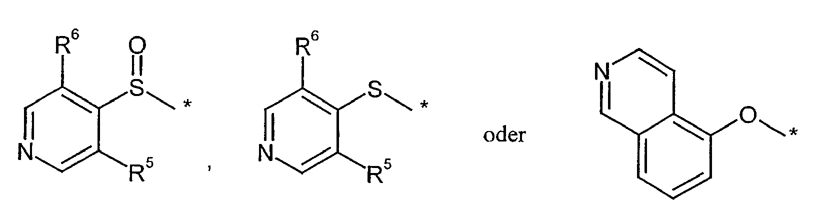 bedeutet, worin
- R⁵ und R⁶: unabhängig voneinander Wasserstoff, Fluor oder Chlor bedeuten,

D
   (1) einen Rest bedeutet, der ausgewählt ist aus der Gruppe von
      Phenyl, das seinerseits durch (C₁-C₄)-Alkylcarbonylamino, Hydroxymethyl, Cyano, (C₁-C₄)-Alkoxymethyl oder 1,2-Dioxymethylen substituiert ist,
      Chinolin, Isochinolin, Indol oder 6-gliedriges Heteroaryl mit 2 oder 3 Stickstoffatomen, wobei die Ringe jeweils über ein Kohlenstoffatom verknüpft sind,
      Pyridylmethyl, 2-Oxo-2H-pyridin-1-yl, 4-Oxo-4H-pyridin-1-yl, die ihrerseits durch Fluor, Chlor oder (C₁-C₄)-Alkyl substituiert sein können, und
      Pyridyl, das seinerseits durch Fluor, Chlor oder (C₁-C₄)-Alkyl substituiert ist,
      oder
   (2) einen Rest *-OR⁷ bedeutet,
      worin
      - R⁷: Phenyl, das durch (C₁-C₄)-Alkyl oder (C₁-C₄)-Alkoxy, die ihrerseits durch Hydroxy und/oder *-NR⁸R⁹ substituiert sein können, Trifluormethyl, Trifluormethoxy, Nitro, Cyano, *-NR⁸R⁹, Fluor, Chlor oder 1,2-Dioxymethylen substituiert sein kann,
      3- bis 7-gliedriges Heterocyclyl mit einem Stickstoffatom, das durch Wasserstoff, (C₁-C₆)-Alkyl oder (C₃-C₈)-Cycloalkyl sub- stituiert sein kann,
      5- oder 6-gliedriges Heteroaryl mit bis zu drei Stickstoff- atomen,
      (C₁-C₆)-Alk-yl oder (C₃-C₇)-Cycloalkyl, die ihrerseits durch Hydroxy oder *-NR⁸R⁹ substituiert sein können,
      Thienyl, Furyl, Pyridylmethyl, Naphthyl oder Benzyl bedeutet,
      worin
      - R⁸ und R⁹: unabhängig voneinander Wasserstoff oder (C₁-C₄)- Alkyl, das seinerseits durch Hydroxy oder Amino sub- stituiert sein kann, bedeuten oder
      - R⁸ und R⁹: gemeinsam mit dem Stickstoffatom, an das sie gebunden sind, einen 5- bis 7-gliedrigen Heterocyclus bilden, der noch ein Sauerstoffatom oder eine Gruppe N-H oder N-(C₁-C₄)-Alkyl im Ring aufweisen kann,
      oder
   (3) einen Rest *-NR¹⁰R¹¹ bedeutet,
      worin
      - R¹⁰: Wasserstoff oder (C₁-C₄)-Alkyl bedeutet und
      - R¹¹: einen Rest *-(CH₂)ₓ-Phenyl, wobei Phenyl bis zu vierfach, unabhängig voneinander, durch Fluor, Chlor oder (C₁-C₄)- Alkyl substituiert sein kann, oder *-(CH₂)_{y}-E bedeutet,
      worin
      - x: 1, 2 oder 3 bedeutet,
      - y: 0, 1, 2 oder 3 bedeutet und
      - E: Pyrrolidin oder Piperidin, die ihrerseits durch (C₁-C₄)- Alkyl substituiert sein können, oder Pyridyl, das bis zu vierfach, unabhängig voneinander, durch Fluor, Chlor oder (C₁-C₄)-Alhyl substituiert sein kann, bedeutet,
      oder
      R¹⁰ und R¹¹ gemeinsam mit dem Stickstoffatom, an das sie gebunden sind, einen 5- oder 6-gliedrigen Heterocyclus bilden, der durch *-NR¹²R¹³, 1,1-Dioxyethylen, 5- oder 6-gliedriges Heterocyclyl mit einem oder zwei Heteroatomen N und/oder O, das seinerseits durch (C₁-C₄)-Alkyl substituiert sein kann, (C₁-C₄)-Alkoxy, durch Hydroxy oder (C₁-C₄)-Alkoxy substituiertes (C₁-C₄)-Alkyl, oder (C₁-C₄)-Alkoxycarbonyl substituiert ist,
      worin
      R¹² und R¹³ unabhängig voneinander Wasserstoff, (C₁-C₆)-Alkyl, (C₁-C₄)-Alkoxycarbonyl, (C₃-C₈)-Cycloalkyl oder (C₁-C₄)-Alkanoyl bedeuten
      oder
      R¹² und R¹³ gemeinsam mit dem Stickstoffatom, an das sie gebunden sind, einen 5- oder 6-gliedrigen Heterocyclus bilden,
      oder
      R¹⁰ und R¹¹ gemeinsam mit dem Stickstoffatom, an das sie gebunden sind, einen 7- bis 12-gliedrigen bicyclischen Heterocyclus bilden, der anneliert oder spirocyclisch ist und ein oder zwei weitere Heteroatomen aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann, oder
      R¹⁰ und R¹¹ gemeinsam mit dem Stickstoffatom, an das sie gebunden sind, einen Rest 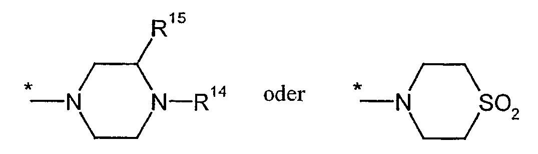 bilden,
      worin
      - R¹⁴: (C₂-C₆)-Alkenyl, (C₁-C₄)-Alkoxycarbonyl, oder *-(CH₂)_{z}-G bedeutet,
      worin
      - z: 0 oder 1 bedeutet und
      - G: (C₃-C₈)-Cycloalkyl, Pyridyl, gegebenenfalls durch (C₁-C₄)-Alkyl oder (C₁-C₄)-Alkoxy sub- stituiertes Phenyl, Tetrahydrofuran oder 1,3- Dioxolan bedeutet,
      und
      - R¹⁵: Wasserstoff oder (C₁-C₄)-Alkyl bedeutet,
   und ihre Salze, Hydrate, Hydrate der Salze und Solvate.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methyhnorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkanoyl, Alkylcarbonylamino, Alkoxycarbonyl und Alkoxymethyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkanoyl steht beispielhaft und vorzugsweise für Acetyl und Propanoyl.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Alkoxycarbonylamino steht beispielhaft und vorzugsweise für Methoxycarbonylamino, Ethoxycarbonylamino, n-Propoxycarbonylamino, Isopropoxycarbonylamino, tert.-Butoxycarbonylamino, n-Pentoxycarbonylamino und n-Hexoxycarbonylamino.

Alkenyl steht für einen linearen oder verzweigten Alkenylrest mit in der Regel 2 bis 6 Kohlenstoffatomen. Bevorzugt ist ein geradkettiger oder verzweigter Alkenylrest mit 2 bis 4, besonders bevorzugt mit 2 oder 3 Kohlenstoffatomen. Beispielsweise und vorzugsweise seien genannt: Vinyl, Allyl, n-Prop-1-en-1-yl und n-But-2-en-1-yl.

Cycloalkyl steht für eine Cycloalkylgruppe mit in der Regel 3 bis 8, bevorzugt 5 bis 7 Kohlenstoffatomen, beispielhaft und vorzugsweise für Cyclopropyl, Cyclobutyl, Cyclopentyl, Cyclohexyl und Cycloheptyl.

Heteroaryl steht für einen aromatischen, mono- oder bicyclischen Rest mit in der Regel 5 bis 10, vorzugsweise 5 oder 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

Heterocyclyl steht für einen mono- oder polycyclischen, vorzugsweise mono- oder bicyclischen heterocyclischen Rest mit in der Regel 4 bis 12, vorzugsweise 5 bis 8 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Im Fall von polycyclischen, beispielsweise bicyclischen Resten kann ein Ring aromatisch sein. Bevorzugt sind 5- bis 8-gliedrige, monocyclische gesättigte Heterocyclylreste mit bis zu zwei Heteroatomen aus der Reihe O, N und S, wie beispielhaft und vorzugsweise Tetrahydrofuran-2-yl, Pyrrolidin-2-yl, Pyrrolidin-3-yl, Pyrrolinyl, Piperidinyl, Morpholinyl, Perhydroazepinyl.

Ein Symbol * an einer Bindung bedeutet die Verknüpfungsstelle im Molekül.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt sind Verbindungen der Formel (I),
worin
- R¹: Amino bedeutet,
- R²: Wasserstoff bedeutet,
- R³ und R⁴: unabhängig voneinander Wasserstoff, Fluor oder Chlor bedeuten,
- A: einen Rest 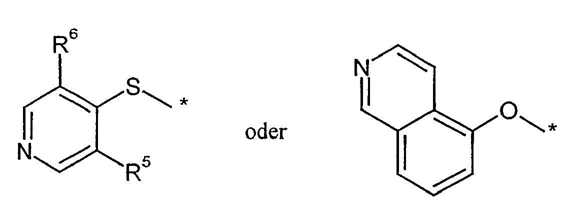 bedeutet,
worin
- R⁵ und R⁶: Wasserstoff bedeuten,
D
(1) einen Rest bedeutet, der ausgewählt ist aus der Gruppe von
   Phenyl, das durch (C₁-C₄)-Alkylcarbonylamino, Hydroxymethyl, (C₁-C₄)-Alkoxymethyl oder 1,2-Dioxymethylen substituiert ist,
   Chinolin, Indol oder 6-gliedriges Heteroaryl mit 2 oder 3 Stickstoffatomen, wobei die Ringe jeweils über ein Kohlenstoffatom verknüpft sind,
   Pyridylmethyl, das durch (C₁-C₄)-Alkyl substituiert sein kann,
   und
   Pyridyl, das durch (C₁-C₄)-Alkyl substituiert ist,
   oder
(2) einen Rest *-OR⁷ bedeutet,
   worin
   R⁷ Phenyl, das durch Fluor, Chlor, (C₁-C₄)-Alkyl, (C₁-C₄)-Alkoxy oder 1,2-Dioxymethylen substituiert sein kann,
   (C₁-C₆)-Alkyl oder (C₃-C₈)-Cycloalkyl, die ihrerseits durch Hydroxy oder *-NR⁸R⁹ substituiert sein können,
   Naphthyl oder Benzyl bedeutet,
   worin
   R⁸ und R⁹ unabhängig voneinander Wasserstoff oder (C₁-C₄)-Alkyl bedeuten
   oder
   R⁸ und R⁹ gemeinsam mit dem Stickstoffatom, an das sie
   gebunden sind, einen 5- bis 7-gliedrigen Heterocyclus bilden, der noch ein Sauerstoffatom oder eine Gruppe N-H oder N-(C₁-C₄)-Alkyl im Ring aufweisen kann,
   oder
(3) einen Rest *-NR¹⁰R¹¹ bedeutet,
   worin
   - R¹⁰: Wasserstoff oder (C₁-C₄)-Alkyl bedeutet und
   - R¹¹: einen Rest *-(CH₂)ₓ-Phenyl, wobei Phenyl bis zu vierfach, unabhängig voneinander, durch Fluor, Chlor oder (C₁-C₄)- Alkyl substituiert sein kann, oder *-(CH₂)_{y}-E bedeutet,
   worin
   x 1 oder 2 bedeutet,
   y 0, 1 oder 2 bedeutet und
   E Pyrrolidin oder Piperidin, die ihrerseits durch (C₁-C₄)- Alkyl substituiert sein können, oder Pyridyl, das bis zu vierfach, unabhängig voneinander, durch Fluor, Chlor oder (C₁-C₄)-Alkyl substituiert sein kann, bedeutet,
   oder
   R¹⁰ und R¹¹ gemeinsam mit dem Stickstoffatom, an das sie gebunden sind, einen 5- oder 6-gliedrigen Heterocyclus bilden, der durch *-NR¹²R¹³, 1,1-Dioxymethylen, 5- oder 6-gliedriges Heterocyclyl mit einem oder zwei Heteroatomen N und/oder O, das seinerseits durch (C₁-C₄)-Alkyl substituiert sein kann, oder (C₁-C₄)-Alkoxymethyl substituiert ist,
   worin
   R¹² und R¹³ unabhängig voneinander Wasserstoff, (C₁-C₆)-Alkyl, (C₃-C₈)-Cycloalkyl oder (C₁-C₄)-Alkanoyl bedeuten oder
   R¹² und R¹³ gemeinsam mit dem Stickstoffatom, an das sie gebunden sind, einen 5- oder 6-gliedrigen Heterocyclus bilden,
   oder
   R¹⁰ und R¹¹ gemeinsam mit dem Stickstoffatom, an das sie gebunden sind, einen 8- bis 10-gliedrigen bicyclischen Heterocyclus bilden, der anneliert oder spirocyclisch ist und ein oder zwei weitere Heteroatomen aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
   oder
   R¹⁰ und R¹¹ gemeinsam mit dem Stickstoffatom, an das sie gebunden sind, einen Rest 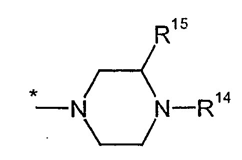 bilden,
   worin
   - R¹⁴: (C₃-C₈)-Cycloalkyl, (C₂-C₆)-Alkenyl, (C₁-C₄)- Alkoxycarbonyl oder Tetrahydrofuran-2-yl-methyl bedeutet,
   und
   - R¹⁵: Wasserstoff oder (C₁-C₄)-Alkyl bedeutet,
und ihre Salze, Hydrate, Hydrate der Salze und Solvate.

Besonders bevorzugt sind Verbindungen der Formel (I),
worin
- R¹: Amino bedeutet,
- R²: Wasserstoff bedeutet,
- R³ und R⁴: unabhängig voneinander Wasserstoff, Fluor oder Chlor bedeuten,
- A: einen Rest 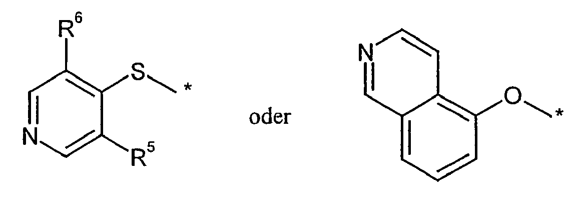 bedeutet,
worin
R⁵ und R⁶ Wasserstoff bedeuten,
D
(1) einen Rest bedeutet, der ausgewählt ist aus der Gruppe von Chinolin, Indol, Pyrazin, Pyridazin und Triazin, wobei die Ringe jeweils über ein Kohlenstoffatom verknüpft sind,
   oder
(2) einen Rest *-OR⁷ bedeutet,
   worin
   - R⁷: Phenyl, das durch Fluor, Chlor, (C₁-C₄)-Alkyl, (C₁-C₄)-Alkoxy oder 1,2-Dioxymethylen substituiert sein kann,
   (C₁-C₆)-Alkyl oder (C₃-C₈)-Cycloalkyl, die ihrerseits durch Hydroxy oder *-NR⁸R⁹ substituiert sein können, bedeutet,
   worin
   R⁸ und R⁹ unabhängig voneinander Wasserstoff oder (C₁-C₄)- Alkyl bedeuten
   oder
   R⁸ und R⁹ gemeinsam mit dem Stickstoffatom, an das sie gebunden sind, einen Morpholin- oder Piperazinring bilden, wobei das zweite Stickstoffatom des Pipera- zinringes durch (C₁-C₄)-Alkyl substituiert sein kann,
   oder
(3) einen Rest *-NR¹⁰R¹¹ bedeutet,
   worin
   - R¹⁰: Wasserstoff oder (C₁-C₄)-Alkyl bedeutet und
   - R¹¹: einen Rest *-(CH₂)_{y}-E bedeutet,
   worin
   y 0 oder 1 bedeutet und
   E Pyrrolidin oder Pyridyl, die ihrerseits durch (C₁-C₄)- Alkyl substituiert sein können, bedeutet,
   oder
   R¹⁰ und R¹¹ gemeinsam mit dem Stickstoffatom, an das sie gebunden sind, einen Pyrrolidin- oder Piperidinring bilden, die durch *-NR¹²R¹³, 1,1-Dioxymethylen, 5- oder 6-gliedriges Heterocyclyl mit einem oder zwei Heteroatomen N und/oder O, das seinerseits durch (C₁-C₄)-Alkyl substituiert sein kann, oder (C₁-C₄)-Alkoxymethyl substituiert ist,
   worin
   R¹² und R¹³ unabhängig voneinander Wasserstoff, (C₁-C₆)-Alkyl, (C₃-C₈)-Cycloalkyl oder (C₁-C₄)-Alkanoyl bedeuten
   oder
   R¹2 und R¹³ gemeinsam mit dem Stickstoffatom, an das sie gebunden sind, einen 5- oder 6-gliedrigen Heterocyclus bilden,
   oder
   R¹⁰ und R¹¹ gemeinsam mit dem Stickstoffatom, an das sie gebunden sind, einen 8- bis 10-gliedrigen bicyclischen Heterocyclus bilden, der anneliert oder spirocyclisch ist und ein oder zwei weitere Heteroatomen aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
und ihre Salze, Hydrate, Hydrate der Salze und Solvate.

Ganz besonders bevorzugt sind Kombinationen von zwei oder mehreren der oben genannten Vorzugsbereiche.

Gegenstand der vorliegenden Erfindung ist auch ein Verfahren zur Herstellung der Verbindungen der Formel (I), das dadurch gekennzeichnet ist, dass man entweder
[A] Verbindungen der Formel (II) 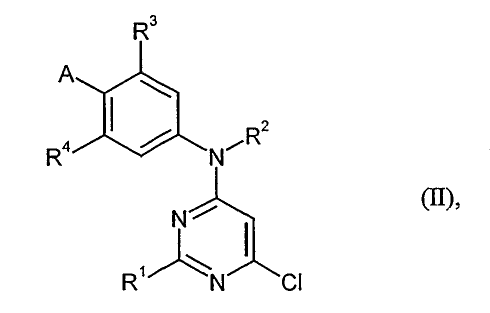 worin
   A, R¹, R², R³ und R⁴ die oben angegebene Bedeutung aufweisen,
   mit Verbindungen der Formel (III)

   D-X¹ (III),

   worin
   - D: die oben angegebene Bedeutung aufweist und
   - X¹: für Wasserstoff oder *-B(OH)₂ steht,
   oder
[B] Verbindungen der Formel (IV) 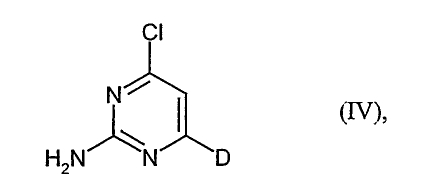 worin
   - D: die oben angegebene Bedeutung aufweist,
   mit Verbindungen der Formel (V) 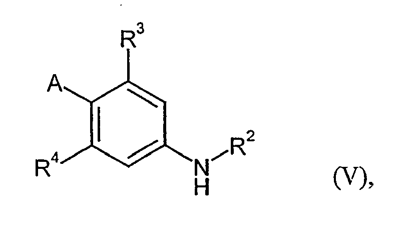 worin
   - A, R², R³ und R⁴: die oben angegebene Bedeutung aufweisen,
zu Verbindungen der Formel (I) umsetzt.

Im Verfahrensschritt [ A ] für den Fall, dass X¹ für Wasserstoff steht, erfolgt die Umsetzung in inerten Lösungsmitteln oder in Substanz, gegebenenfalls in Gegenwart einer Base, bevorzugt in einem Temperaturbereich von 20°C bis zum Rückfluss der Lösungsmittel oder in der Schmelze bei Normaldruck.

Inerte Lösungsmittel sind beispielsweise Alkohole wie Methanol, Ethanol, Propanol, Isopropanol oder Butanol, *N*-alkylierte Carbonsäureamide wie Dimethylformamid oder Dimethylacetamid, Alkylsulfoxide wie Dimethylsulfoxid, oder andere Lösungsmittel wie Acetonitril oder Pyridin, bevorzugt sind Ethanol oder Dimethylformamid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt Diisopropylethylamin oder Triethylamin.

Im Verfahrensschritt [A] für den Fall, dass X¹für *-B(OH)₂ steht, erfolgt die Umsetzung zu Verbindungen der Formel (I) im Allgemeinen in inerten Lösungsmitteln, in Gegenwart eines Übergangsmetallkatalysators, in Gegenwart einer Base, bevorzugt in einem Temperaturbereich von 70°C bis 110°C bei Normaldruck.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, Nitroaromaten wie Nitrobenzol, gegebenenfalls *N*-alkylierte Carbonsäureamide wie Dimethylformamid, Dimethylacetamid, Alkylsulfoxide wie Dimethylsulfoxid oder cyclische Lactame wie *N*-Methylpyrrolidon. Die Lösungsmittel finden gegebenenfalls unter Zusatz von Ethanol Verwendung. Bevorzugt sind Lösungsmittel aus der Reihe Dimethylformamid, 1,2-Dimethoxyethan und Toluol/Ethanol.

Als Übergangsmetallkatalysatoren werden bevorzugt Palladium(0)- oder Palladium-(II)-verbindungen, insbesondere Bis-(diphenylphosphanferrocenyl)-palladium(II)-chlorid, Dichlorbis(triphenylphosphin)-palladium oder Tetrakis(triphenylphosphin)-palladium(0) verwendet.

Als Basen werden Kalium-tert.-butylat, oder Alkalihydroxide oder -salze wie Kaliumacetat, Natriumhydroxid, Natriumhydrogencarbonat, Natriumcarbonat oder Kaliumcarbonat, gegebenenfalls in Form ihrer wässrigen Lösungen, bevorzugt.

Im Verfahrensschritt [B] erfolgt die Umsetzung zu Verbindungen der Formel (I) in konzentrierter Salzsäure, bevorzugt in einem Temperaturbereich von 70°C bis 110°C bei Normaldruck. Bei dieser Umsetzung kann die Amino-Gruppe am Pyrimidin gegebenenfalls zur Hydroxy-Gruppe hydrolysiert werden.

Zur Herstellung der Verbindungen der Formel (II) aus Verfahrensschritt [A] setzt man Verbindungen der Formel (V) mit der Verbindung der Formel (VI) 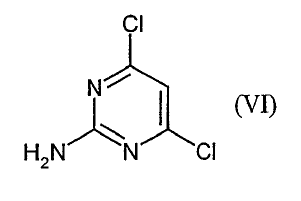 unter Reaktionsbedingungen, wie für den Verfahrensschritt [B] beschrieben, um.

Bei dieser Umsetzung kann die Amino-Gruppe am Pyrimidin gegebenenfalls zur entsprechenden Hydroxy-Gruppe hydrolysiert werden.

Zur Herstellung der Verbindungen der Formel (IV) aus Verfahrensschritt [B] setzt man Verbindungen der Formel (VII) 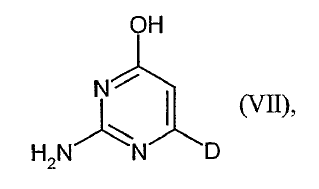 worin
- D: die oben angegebene Bedeutung aufweist,
mit Phosphorylchlorid in N,N-Dimethylanilin, bevorzugt in einem Temperaturbereich von 70°C bis 110°C bei Normaldruck um.

In einer anderen Verfahrensvariante setzt man zur Herstellung der Verbindungen der Formel (IV) Verbindungen der Formel (VI) mit Verbindungen der Formel (III) unter Reaktionsbedingungen, wie für den Verfahrensschritt [A] beschrieben, um.

Zur Herstellung der Verbindungen der Formel (VII) setzt man Verbindungen der Formel (VIII) 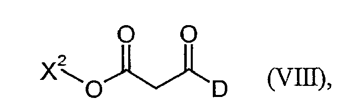 worin
- D: die oben angegebene Bedeutung aufweist und
- X²: für Alkyl, bevorzugt für Methyl oder Ethyl, steht,
mit der Verbindung der Formel (IX) 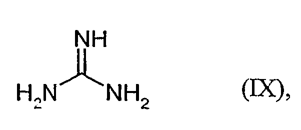 um.

Die Umsetzung der Verbindungen der Formel (VIII) und (IX) erfolgt zunächst mit konzentrierter Salzsäure in Ethanol, bevorzugt in einem Temperaturbereich von 50°C bis zum Rückfluss der Lösungsmittel bei Normaldruck, und anschließend mit wässriger Natronlauge, bevorzugt in einem Temperaturbereich von 50°C bis zum Rückfluss der Lösungsmittel bei Normaldruck.

Zur Herstellung von Verbindungen der Formel (Va) aus Verfahrensschritt [B], in welcher R² für (C₁-C₆)-Alkyl oder (C₃-C₈)-Cycloalkyl steht, setzt man Verbindungen der Formel (Vb), in welcher R² für Wasserstoff steht,
mit Verbindungen der Formel (X)

R²-X⁴ (X),

worin
- R²: für (C₁-C₆)-Alkyl oder (C₃-C₈)-Cycloalkyl und
- X⁴: für Halogen, bevorzugt Brom oder Chlor, steht,
um.

Die Umsetzung erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlorethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt Tetrahydrofuran, Methylenchlorid, Aceton, 2-Butanon, Acetonitril, Dimethylformamid oder 1,2-Dimethoxyethan.

Basen sind beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.-butylat, oder Amide wie Natriumamid, Lithium-bis-(trimethylsilyl)amid oder Lithiumdiisopropylamid, oder metallorganische Verbindungen wie Butyllithium oder Phenyllithium, oder andere Basen wie Natriumhydrid, DBU, bevorzugt Kalium-tert.-butylat, Cäsiumcarbonat, DBU, Natriumhydrid, Kaliumcarbonat oder Natriumcarbonat.

Zur Herstellung der Verbindungen der Formel (Vb) aus Verfahrensschritt [B], in welcher R² für Wasserstoff steht, setzt man Verbindungen der Formel (XI), 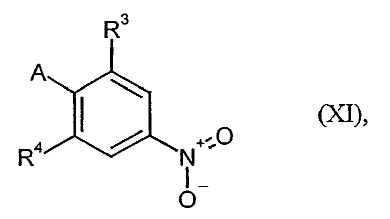 worin
- A, R³ und R⁴: die oben angegebene Bedeutung aufweisen,
mit Reduktionsmitteln um.

Die Umsetzung erfolgt im Allgemeinen in inerten Lösungsmitteln, gegebenenfalls in Gegenwart von Hydrazinhydrat, bevorzugt in einem Temperaturbereich von Raumtemperatur bis zum Rückfluss der Lösungsmittel bei Normaldruck bis 3 bar.

Reduktionsmittel sind beispielsweise Palladium auf Aktivkohle und Wasserstoff, Platinoxid auf Aktivkohle und Wasserstoff, Zinndichlorid oder Titantrichlorid, bevorzugt ist Palladium auf Aktivkohle und Wasserstoff in Gegenwart von Hydrazinhydrat oder Platinoxid auf Aktivkohle und Wasserstoff.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, isoPropanol, n-Butanol, tert.-Butanol oder 2-Ethylhexanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Ethanol, n-Butanol oder 2-Ethylhexanol.

Zur Herstellung der Verbindungen der Formel (XI) setzt man Verbindungen der Formel (XII), 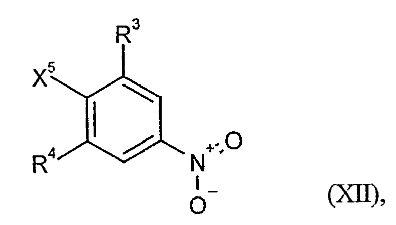 worin
- R³ und R⁴: die oben angegebene Bedeutung aufweisen und
- X⁵: für Halogen, bevorzugt Fluor oder Chlor, steht,
mit Verbindungen der Formel (XIII)

A-H (XIII),

worin
- A: die oben angegebene Bedeutung aufweist,
um.

Die Umsetzung erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt Acetonitril, Dimethylformamid oder 1,2-Dimethoxyethan.

Basen sind beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.-butylat, oder Amide wie Natriumamid, Lithium-bis-(trimethylsilyl)amid oder Lithiumdiisopropylamid, oder metallorganische Verbindungen wie Butyllithium oder Phenyllithium, oder andere Basen wie Natriumhydrid, DBU, bevorzugt Kalium-tert.-butylat, Cäsiumcarbonat, Kaliumcarbonat oder Natriumcarbonat.

Die Verbindungen der Formel (III), (VI), (VIII), (IX), (X), (XII) und (XIII) sind dem Fachmann an sich bekannt oder lassen sich nach üblichen literaturbekannten Verfahren herstellen.

Die Verbindungen der Formel (I) lassen sich beispielsweise durch Umsetzung mit Oxidationsmitteln weiter derivatisieren.

Die Herstellung der erfindungsgemäßen Verbindungen kann durch folgende Syntheseschemata verdeutlicht werden.

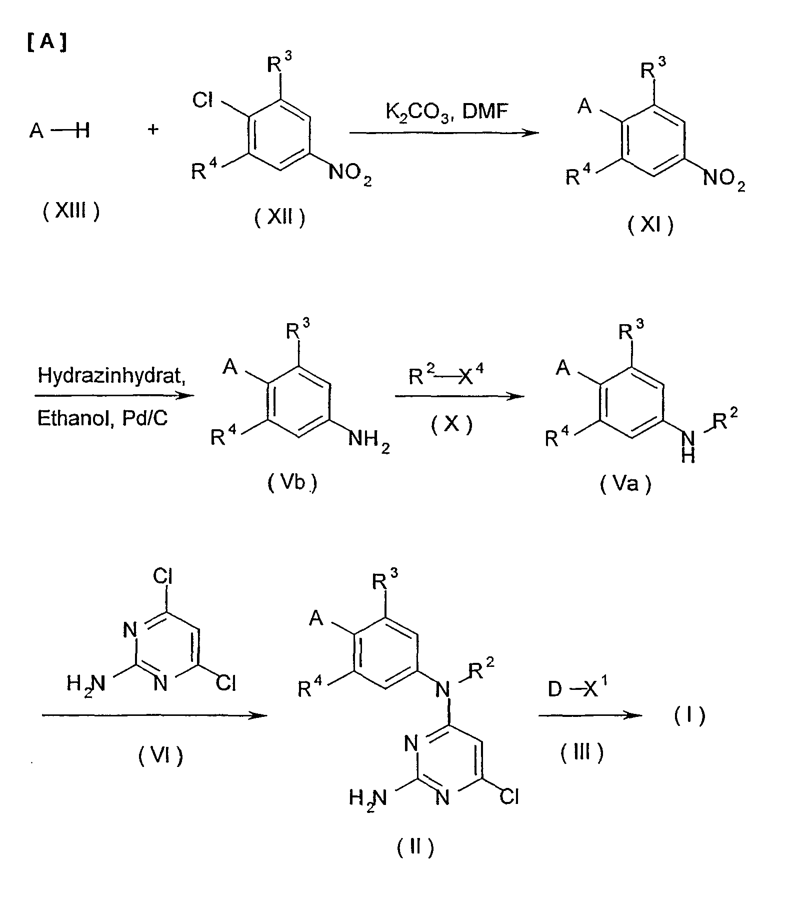

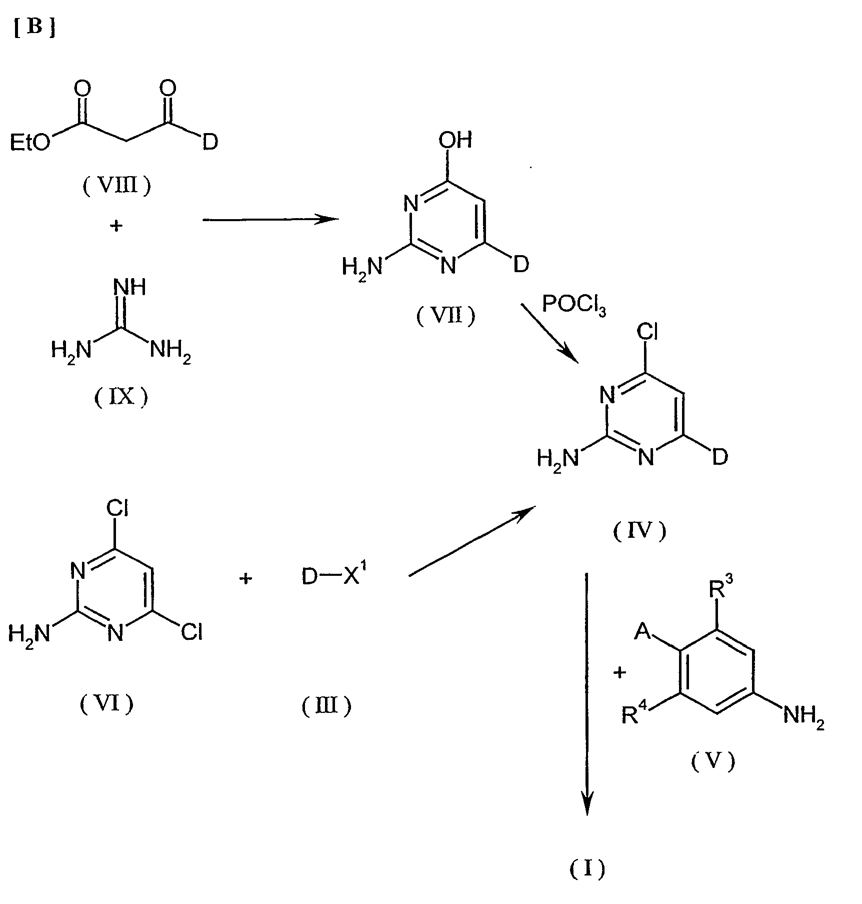

Die erfindungsgemäßen Verbindungen zeigen ein nicht vorhersehbares, wertvolles pharmakologisches und pharmakokinetisches Wirkspektrum. Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen der Formel (I) lässt sich durch ihre Wirkung als Rho-Kinase-Inhibitoren erklären.

Die erfindungsgemäßen Verbindungen der Formel (I) können aufgrund ihrer pharnlakologischen Eigenschaften allein oder in Kombination mit anderen Wirkstoffen eingesetzt werden zur Behandlung und/oder Prophylaxe von Erkrankungen, insbesondere von kardiovaskulären Erkrankungen.

Die Verbindungen der Formel (I) sind geeignet für die Prophylaxe und/oder Behandlung von kardiovaskulären Erkrankungen wie beispielsweise Bluthochdruck und Herzinsuffizienz, stabiler und instabiler Angina pectoris, peripheren und kardialen Gefäßerkrankungen, von Arrhythmien, von thromboembolischen Erkrankungen und Ischämien wie Myokardinfarkt, Hirnschlag, transitorischen und ischämischen Attacken, peripheren Durchblutungsstörungen, Subarachnoidalblutungen, Verhinderung von Restenosen wie beispielsweise nach Thrombolysetherapien, percutanen transluminalen Angioplastien (PTA), percutanen transluminalen Koronarangioplastien (PTCA), Bypass sowie zur Prophylaxe und/oder Behandlung von Arteriosklerose, asthmatischen Erkrankungen, COPD und Krankheiten des Urogenitalsystems wie beispielsweise Prostatahypertrophie, erektiler Dysfunktion, weiblicher sexueller Dysfunktion, Osteoporose, Gastroparese und Inkontinenz.

Weiterhin können die Verbindungen der Formel (I) zur Prophylaxe und/oder Behandlung von Krebserkrankungen, insbesondere von Tumoren eingesetzt werden.

Im Rahmen der vorliegenden Erfindung umfasst die Definition von Tumoren sowohl benigne, wie auch maligne Tumore und damit beispielsweise auch benigne Neoplasien, Dysplasien, Hyperplasien, wie auch Neoplasien mit Metastasenbildung. Weitere Beispiele für Tumore sind Karzinome, Sarkome, Karzinosarkome, Tumore der blutbildenden Organe, Tumore des Nervengewebes z.B. des Gehirns oder Tumore von Hautzellen. Bei der Tumorbildung kommt es zur unkontrollierten oder unzureichend kontrollierten Zellteilung. Der Tumor kann örtlich begrenzt sein, er kann aber auch das umliegende Gewebe infiltrieren und sich dann durch das lymphatische System oder durch den Blutstrom an einem neuen Ort festsetzen. Somit gibt es primäre und sekundäre Tumore. Primäre Tumore sind ursprünglich in dem Organ entstanden, in dem sie gefunden werden. Sekundäre Tumore haben sich durch Metastasenbildung in einem anderen Organ festgesetzt und sich dann an ihrem neuen Ort ausgebreitet.

Weiterer Gegenstand der vorliegenden Erfindung ist die Verwendung der Verbindungen der Formel (I) zur Herstellung von Arzneimitteln zur Prophylaxe und/oder Behandlung der zuvor genannten Krankheitsbilder.

Weiterer Gegenstand der vorliegenden Erfindung ist ein Verfahren zur Prophylaxe und/oder Behandlung der zuvor genannten Krankheitsbilder mit den Verbindungen der Formel (I).

Weiterer Gegenstand der vorliegenden Erfindung sind Arzneimittel, die mindestens eine erfindungsgemäße Verbindung, vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch, als Stents oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nicht überzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und / oder Geruchskorrigentien.

Im Allgemeinen hat es sich sowohl in der Human- als auch in der Veterinärmedizin als vorteilhaft erwiesen, den erfindungsgemäßen Wirkstoff in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoff vorzugsweise in Mengen von etwa 0,1 bis etwa 80, insbesondere 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilern.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- DC: Dünnschichtchromatographie
- DCI: direkt chemische Ionisation (bei MS)
- DCM: Dichlormethan
- DIEA: *N,N*-Diisopropylethylamin
- DMSO: Dimethylsulfoxid
- DMF: *N,N*-Dimethylformamid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- h: Stunde
- HPLC: Hochdruck-, Hochleistungsflüssigchromatographie
- konz.: konzentriert
- LC-MS: Flüssigchromatographie-gekoppelte Massenspektroskopie
- LDA: Lithium-Diisopropylamid
- MPLC: Mitteldruck-, Mittelleistungsflüssigchromatographie
- MS: Massenspektroskopie
- NMR: Kernresonanzspektroskopie
- proz.: prozentig
- RP-HPLC: Reverse Phase HPLC
- RT: Raumtemperatur
- R_{f}: Retentionsindex (bei DC)
- Rₜ: Retentionszeit (bei HPLC)
- THF: Tetrahydrofuran

### HPLC-, LCMS- und GCMS-Methoden:

### Methode 1 (HPLC):

Instrument: HP 1100 mit DAD-Detektion; Säule: Kromasil RP-18, 60 mm x 2 mm, 3.5 µm; Eluent: A=5 ml HClO₄/l Wasser, B=Acetonitril; Gradient: 0 min 2 % B, 0.5 min 2 % B, 4.5 min 90 % B, 6.5 min 90 % B; Fluss: 0.75 ml/min; Temp.: 30°C; Detektion UV 210 nm.

### Methode 2 (HPLC):

Instrument: HP 1100 mit DAD-Detektion; Säule: Kromasil RP-18, 60 mm x 2 mm, 3.5 µm; Eluent: A=5 ml HClO₄/l Wasser, B=Acetonitril; Gradient: 0 min 2 % B, 0.5 min 2 % B, 4.5 min 90 % B, 9 min 90 % B; Fluss: 0.75 ml/min; Temp.: 30°C; Detektion UV 210 nm.

### Methode 3 (HPLC):

Instrument: Finnigan MAT 900S, TSP: P4000,AS3000,UV3000HR; Säule: Symmetry C 18, 150 mm x 2.1 mm, 5.0 µm; Eluent C: Wasser, Eluent B: Wasser + 0.3 g 35 %ige Salzsäure, Eluent A: Acetonitril; Gradient: 0.0 min 2 % A → 2.5 min 95 % A → 5 min 95 % A; Ofen: 70°C; Fluss: 1.2 ml/min; UV-Detektion: 210 nm.

### Methode 4 (LCMS):

Instrument: Micromass Quattro LCZ, HP1100; Säule: Symmetry C18, 50 mm x 2.1 mm, 3.5 µm; Eluent A: Acetonitril + 0.1 % Ameisensäure, Eluent B: Wasser + 0.1 % Ameisensäure; Gradient: 0.0 min 10 % A → 4.0 min 90 % A → 6.0min 90 % A; Ofen: 40°C; Fluss: 0.5 ml/min; UV-Detektion: 208-400 nm.

### Methode 5 (LCMS):

Instrument: Micromass Platform LCZ, HP1100; Säule: Symmetry C18, 50 mm x 2.1 mm, 3.5 µm; Eluent A: Acetonitril + 0.1 % Ameisensäure, Eluent B: Wasser + 0.1 % Ameisensäure; Gradient: 0.0 min 10 % A → 4.0 min 90 % A → 6.0min 90 % A; Ofen: 40°C; Fluss: 0.5 ml/min; UV-Detektion: 208-400 nm.

### Methode 6 (LCMS):

Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05 % Ameisensäure, Eluent A: Wasser + 0.05 % Ameisensäure; Gradient: 0.0 min 10 % B → 3.5 min 90 % B → 5.5 min 90 % B; Ofen: 50°C; Fluss: 0.8 ml/min; UV-Detektion: 210 nm.

### Methode 7 (LCMS):

Instrument: Micromass Platform LCZ, HP1100; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 90 % A → 4.0 min 10 % A → 6.0 min 10 % A; Ofen: 40°C; Fluss: 0.5 ml/min; UV-Detektion: 208-400 nm.

### Methode 8 (LCMS):

Instrument: Micromass Quattro LCZ, HP1100; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 90 % A → 4.0 min 10 % A → 6.0 min 10 % A; Ofen: 40°C; Fluss: 0.5 ml/min; UV-Detektion: 208-400 nm.

### Methode 9 (GCMS):

Säule: HP-5 30 m x 320 µm x 0.25 µm (Filmdicke); Trägergas: Helium; Temperaturgradient: 14°C/min bis 300°C, dann 1 min konst. 300°C; Fluss: 1.5 ml/min; Anfangstemperatur: 60°C; Anfangszeit: 2 min; Frontinjektor-Temp.: 250°C.

### Methode 10 (HPLC):

Instrument: Waters Alliance 2790 LC; Säule: Symmetry C18, 50 mm x 2.1, 3.5 µm; Eluent A: Wasser + 0.1 % Ameisensäure, Eluent B: Acetonitril + 0.1 % Ameisensäure; Gradient: 0.0 min 5 % B → 5.0 min 10 % B → 6.0 min 10 % B; Temperatur: 50°C; Fluss: 1.0 ml/min; UV-Detektion: 210 nm.

### Methode 11 (chirale HPLC):

Säule: chirale stationäre Phase, basierend auf dem optisch aktiven Monomer N-Methacrylacyl-L-leucin-dicyclopropylmethylamid; Eluent A: iso-Hexan, Eluent B: Essigsäureethylester; Gradient: A:B → 20:80; Fluss: 15 ml/min.

### Methode 12 (LCMS):

Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50 %ige Ameisensäure, Eluent B: 1 1 Acetonitril + 1 ml 50 %ige Ameisensäure; Gradient: 0.0 min 100 % A → 0.2 min 100% A → 2.9 min 30 % A → 3.1 min 10 % A → 4.5 min 10 % A; Ofen: 55°C; Fluss: 0.8 ml/min; UV-Detektion: 208-400 nm.

### Methode 13 (LCMS):

Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05 % Ameisensäure, Eluent A: Wasser + 0.05 % Ameisensäure; Gradient: 0.0 min 5 % B → 2.0 min 40 % B → 4.5 min 90 % B→ 5.5 min 90 % B; Ofen: 45 °C; Fluss: 0.0 min 0.75 ml/min → 4.5 min 0.75 ml/min→ 5.5 min 1.25 ml/min; UV-Detektion: 210 nm.

### Methode 14 (LCMS):

Instrument: Micromass Quattro LCZ mit HPLC Agilent Serie 1100; Säule: UPTISPHERE HDO, 50 mm x 2.0 mm, 3 µm; Eluent A: 1 1 Wasser + 1 ml 50 %ige Ameisensäure, Eluent B: 1 1 Acetonitril + 1 ml 50 %ige Ameisensäure; Gradient: 0.0 min 100 % A → 0.2 min 100 % A → 2.9 min 30 % A → 3.1 min 10 % A → 4.5 min 10 % A; Ofen: 55°C; Fluss: 0.8 ml/min; UV-Detektion: 208-400 nm.

### Methode 15 (LCMS):

Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05 % Ameisensäure, Eluent A: Wasser + 0.05 % Ameisensäure; Gradient: 0.0 min 5 % B → 4.5 min 90 % B → 5.5 min 90 % B; Ofen: 50 °C; Fluss: 1.0 ml/min; UV-Detektion: 210 nm.

### Ausgangsverbindungen

### Beispiel I

### 4-Chlor-6-(1H-indol-5-yl)-2-pyrimidinamin

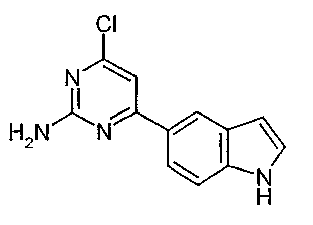

380 mg (2.33 mmol) 2-Amino-4,6-dichlorpyrimidin werden in 20 ml Toluol und 10 ml Ethanol suspendiert und mit 80 mg (0.07 mmol) Tetrakis(triphenylphosphin)palladium(0) versetzt. Nach 20 Minuten Rühren bei Raumtemperatur werden 450 mg (2.80 mmol) 5-Indolboronsäure und 3.90 ml einer 2M Natriumcarbonatlösung zugegeben. Man lässt 20 Stunden bei 120°C rühren. Nach dem Abkühlen wird die Reaktionslösung mit 1N Salzsäure neutral gestellt und dreimal mit je 50 ml Ethylacetat extrahiert. Die organische Phase wird über Natriumsulfat getrocknet, abfiltriert und im Vakuum eingeengt. Das Rohprodukt wird über Kieselgel 60 chromatographisch gereinigt (Laufmittel: Cyclohexan → Cyclohexan-Ethylacetat 1:1).

Man erhält 32 mg (4 % d. Th.) Produkt.

¹H-NMR (400 MHz, DMSO-d₆): δ = 6.47 (s, 1H), 7.33 (s, 1H), 7.50 (dd, 2H), 7.98 (s, 1H), 11.13 (s, 1H)
MS (ESIpos): m/z = 245 (M+H)⁺
HPLC (Methode 1): Rₜ = 4.12 min

Das in der folgenden Tabelle aufgeführte Beispiel kann analog der für Beispiel I beschriebenen Vorschrift aus den entsprechenden Ausgangsverbindungen hergestellt werden.

| **Beispiel** | **Struktur** | **Analytische Daten** |
|---|---|---|
| **II** | 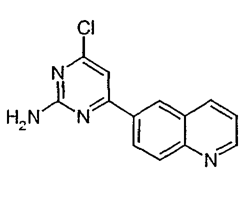 | ¹H-NMR (300 MHz, DMSO-d₆): δ = 7.24 (br.s, 2H), 7.44 (s, 1H), 7.61 (dd, 1H), 8.12 (d, 1H), 8.45 (dt, 2H), 8.77 (d, 1H), 8.98 (dd, 1H) |
| | | MS (ESIpos): m/z = 257 (M+H)⁺ |
| | | HPLC (Methode 1): Rₜ = 3.54 min |

### Beispiel III

### 3-Oxo-3-(4-pyridinyl)propansäureethylester

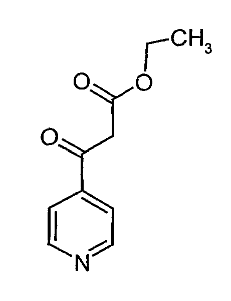

25 g (203 mmol) Isonicotinsäure, 35.12 g (243.7 mmol) 2,2-Dimethyl-1,3-dioxolan-4,6-dion und 49.6 g (406 mol) 4-Dimethylaminopyridin werden in 300 ml Dichlormethan vorgelegt und auf 0°C gekühlt. Es wird eine 1N Lösung von 46.1 g (223.4 mmol) 1,3-Dicyclohexylcarbodiimid in Dichlormethan zugetropft. Es wird 2 Stunden bei Raumtemperatur nachgerührt. Der entstandene Niederschlag wird abfiltriert und mit Dichlormethan nachgewaschen. Das Filtrat wird im Vakuum eingeengt. Der Rückstand wird in 1200 ml Ethanol gelöst und mit einer Lösung aus 96.6 g (507.7 mmol) p-Toluolsulfonsäure-monohydrat in 300 ml Ethanol versetzt und eine Stunde unter Rückfluss gerührt. Nach dem Abkühlen wird das Ethanol im Vakuum abgezogen. Der Rückstand wird in 1000 ml Ethylacetat und 900 ml Wasser aufgenommen und in der Hitze gelöst. Die organische Phase wird abgetrennt, mit 600 ml gesättigter Natriumhydrogencarbonatlösung und gesättigter Natriumchloridlösung gewaschen und über Natriumsulfat getrocknet. Es wird im Vakuum eingeengt. Das Rohprodukt wird über eine Kieselgel-Fritte mit Dichlormethan-Methanol 10:1 filtriert. Da die wässrige Phase noch Produkt enthält, wird diese mit Dichlormethan extrahiert, über Natriumsulfat getrocknet und im Vakuum eingeengt. Das Rohprodukt wird über eine Kieselgel-Fritte mit Dichlormethan-Methanol 10:1 filtriert.

Insgesamt erhält man 25.9 g (42 % d. Th.) Produkt.

¹H-NMR (300 MHz, DMSO-d₆): δ = 1.17 (t, 3H), 4.12 (q, 2H), 4.25 (s, 2H), 7.82 (dd, 2H), 8.83 (dd, 2H)
LC-MS (Methode 3): Rₜ = 2.40 min
MS (ESIpos): m/z = 194 (M+H)⁺

### Beispiel IV

### 2-Amino-6-(4-pyridinyl)-4-pyrimidinol

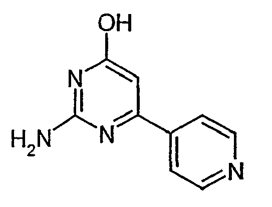

25 g (81.52 mmol) der Verbindung aus Beispiel III und 13.22 g (73.37 mmol) Guanidiniumcarbonat werden in 250 ml Ethanol gelöst, mit konzentrierter Salzsäure versetzt und über Nacht unter Rückfluss gerührt. Nach dem Abkühlen wird der Niederschlag abgesaugt, mit Ethanol nachgewaschen und im Hochvakuum getrocknet. Der Feststoff wird mit 250 ml 1N Natriumhydroxidlösung versetzt und 2 Stunden unter Rückfluss gerührt. Nach dem Abkühlen wird mit konzentrierter Essigsäure sauer gestellt, das ausgefallene Produkt abgesaugt und mit Diethylether nachgewaschen.

Nach dem Trocknen im Hochvakuum erhält man 12.52 g (82 % d. Th.) Produkt.

¹H-NMR (300 MHz, DMSO-d₆): δ = 6.23 (s, 1H), 6.89 (br.s, 2H), 7.86 (dd, 2H), 8.64 (dd, 2H), 11.65 (br.s, 1H).
LC-MS (Methode 4): Rₜ = 0.30 min
MS (ESIpos): m/z =189 (M+H)⁺

### Beispiel V

### 4-Chlor-6-(4-pyridinyl)-2-pyrimidinamin

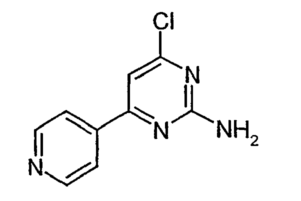

10.4 g (55.26 mmol) der Verbindung aus Beispiel IV werden in 28.33 ml (303.95 mmol) Phosphorylchlorid gelöst. Es werden 0.88 g (7.18 mmol) N,N-Dimethylanilin langsam zugetropft und eine Stunde bei 100°C gerührt. Anschließend wird die Reaktionslösung noch 2 Stunden bei Raumtemperatur gerührt. Das Phosphorylchlorid wird im Vakuum abrotiert. Der Rückstand wird mit Wasser-Dichlormethan 9:1 versetzt und für 5 Minuten aufgekocht. Dann wird mit gesättigter Natriumhydrogencarbonatlösung neutralisiert, das Produkt abgesaugt und im Hochvakuum getrocknet.

Es werden 5.9 g (49 % d. Th.) Produkt erhalten.

¹H-NMR (300 MHz, DMSO-d₆): δ = 7.31 (br.s, 2H), 7.38 (s, 1H), 8.00 (dd, 2H), 8.74 (dd, 2H)
LC-MS (Methode 4): Rₜ = 1.08 min
MS (ESIpos): m/z = 207 (M+H)⁺

### Beispiel VI

### 1-Chlor-2,3-difluor-5-nitrobenzol

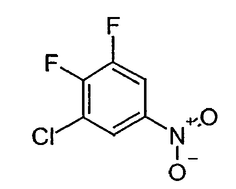

Die Verbindung ist zugänglich durch Oxidation des in JP 05059067 beschriebenen 3-Chlor-4,5-difluoranilins mit Wasserstoffperoxid in Trifluoressigsäure gemäß einem Verfahren, das zur Herstellung analoger Derivate beschrieben ist in Heaton, A. et al., J. Fluorine Chem. 1997, 81 (2), 133-138 und Krapcho, A. P. et al., J. Org. Chem. 1990, 55 (21), 5662-5664.

### Beispiel VII

### 4-[(2-Fluor-4-nitrophenyl)sulfanyl]pyridin

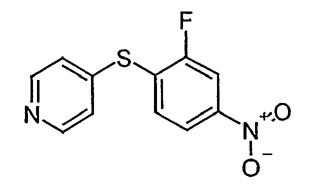

21 g (188.9 mmol) 4-Mercaptopyridin, 30.05 g (188.9 mmol) 3,4-Difluornitrobenzol und 60.05 g (434.5 mmol) Kaliumcarbonat werden in Dimethylformamid gelöst und 3 Stunden bei 40°C gerührt. Anschließend wird die Reaktionslösung mit 500 ml Ethylacetat und 300 ml Wasser verdünnt. Die wässrige Phase wird fünfmal mit je 100 ml Ethylacetat extrahiert. Die vereinigten organischen Phasen werden mit 200 ml gesättigter Natriumchloridlösung gewaschen, über Natriumsulfat getrocknet und im Vakuum einrotiert. Der Rückstand wird über eine MPLC gereinigt (Laufmittel: Ethylacetat-Cyclohexan 1:1)

Es werden 37.3 g (79 % d. Th.) Produkt erhalten.

¹H-NMR (300 MHz, DMSO-d₆): δ = 7.28 (dd, 2H), 7.79 (t, 1H), 8.15 (dd, 1H), 8.30 (dd, 1H), 8.50 (dd, 2H)
LC-MS (Methode 4): Rₜ = 2.68 min
MS (ESIpos): m/z = 251 (M+H)⁺

Die in der folgenden Tabelle aufgeführten Beispiele können analog der für Beispiel VII beschriebenen Vorschrift aus den entsprechenden Mercapto- bzw. Hydroxyheterocyclen und deren entsprechenden 4-Fluor- bzw. 4-Chlornitrobenzol-Derivaten hergestellt werden.

| **Beispiel** | **Struktur** | **Analytische Daten** |
|---|---|---|
| **VIII** | 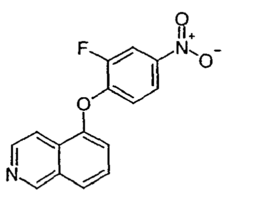 | ¹H-NMR (200 MHz, DMSO-d₆): δ = 7.14 (t, 1H), 7.55 (d, 1H), 7.74 (t, 1H), 7.86 (d, 1H), 8.08 (t, 2H), 8.43 (dd, 1H), 8.58 (d, 1H), 9.46 (s, 1H) |
| | | HPLC (Methode 1): Rₜ = 3.80 min |
| **IX** | 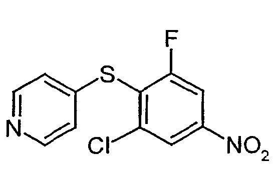 | ¹H-NMR (300 MHz, DMSO-d₆): δ = 7.15 (dd, 2H), 8.37 (dd, 1H), 8.41- 8.45 (m, 3H) |
| | | HPLC (Methode 1 ): Rₜ = 3.77 min |
| | | MS (CIpos): m/z = 302 (M+NH₄)⁺ |
| **X** | 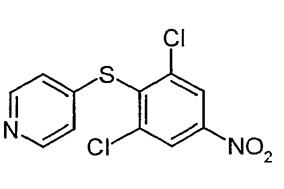 | ¹H-NMR (200 MHz, DMSO-d₆): δ = 7.07 (dd, 2H), 8.41 (dd, 2H), 8.54 (s, 2H) |
| | | HPLC (Methode 1): Rₜ = 3.92 min |
| | | MS (ESIpos): m/z = 301 (M+H)+ |

### Beispiel XI

### 3-Fluor-4-(4-pyridinylsulfanyl)anilin

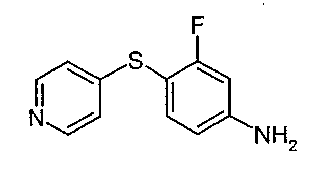

37 g (147.9 mmol) der Verbindung aus Beispiel VII werden in 1000 ml Ethanol gelöst und mit 143.86 ml (2.95 mol) Hydrazinhydrat und 4 g Palladium auf Kohle versetzt. Das Reaktionsgemisch wird über Nacht unter Rückfluss gerührt. Nach dem Abkühlen wird der Ansatz über Kieselgel abgesaugt und mit Ethanol nachgewaschen. Das Filtrat wird im Vakuum einrotiert. Der Rückstand wird mit Diethylether aufgeschlämmt und abgesaugt. Anschließend wird der Niederschlag mit Wasser aufgeschlämmt und abgesaugt. Es wird noch zweimal mit wenig Wasser nachgewaschen.

Nach dem Trocknen im Hochvakuum erhält man 27.3 g (84 % d. Th.) Produkt.

¹H-NMR (300 MHz, DMSO-d₆): δ = 6.02 (br.s, 2H), 6.49-6.54 (m, 2H), 6.93 (dd, 2H), 7.23 (t, 1H), 8.32 (dd, 2H)
LC-MS (Methode 4): Rₜ = 0.96 min
MS (ESIpos): m/z = 221 (M+H)⁺

Das in der folgenden Tabelle aufgeführte Beispiel kann analog der für Beispiel XI beschriebenen Vorschrift aus der Verbindung aus Beispiel VIII hergestellt werden.

| **Beispiel** | **Struktur** | **Analytische Daten** |
|---|---|---|
| **XII** | 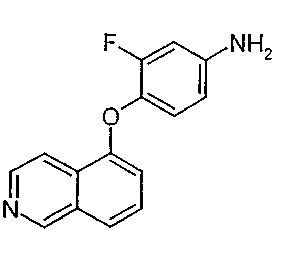 | ¹H-NMR (200 MHz, DMSO-d₆): δ = 5.46 (br.s, 2H), 6.46 (d, 1H), 6.55 (dd, 1H), 6.85 (d, 1H), 7.04 (t, 1H), 7.54 (t, 1H), 7.77 (d, 1H), 8.10 (d, 1H), 8.58 (d, 1H), 9.35 (s, 1H) |
| | | LC-MS (Methode 4): Rₜ = 1.95 min |
| | | MS (ESIpos): m/z = 255 (M+H)⁺ |

### Beispiel XIII

### 3-Chlor-5-fluor-4-(4-pyridinylsulfanyl)anilin

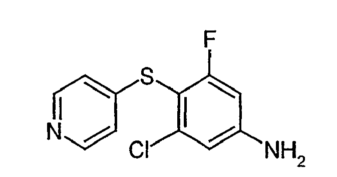

3.19 g (11.205 mmol) der Verbindung aus Beispiel IX werden in 200 ml Ethanol gelöst. Anschließend werden 638 mg (2.81 mmol) Platin(IV)oxid hinzugefügt und der Ansatz wird 2 Stunden bei RT und Normaldruck unter Wasserstoffatmosphäre gerührt. Zur Aufarbeitung wird die Reaktionslösung über Kieselgur abgesaugt und gut mit Ethanol nachgewaschen. Das Filtrat wird im Vakuum eingeengt.

Es werden 2.755 g (81 % d. Th.) Produkt erhalten.

¹H-NMR (200 MHz, DMSO-d₆): δ = 6.37 (s, 2H), 6.49 (dd, 1H), 6.72-6.74 (m, 1H), 6.93 (dd, 2H), 8.34 (dd, 2H)
HPLC (Methode 1): Rₜ = 3.68 min
MS (ESIpos): m/z = 255 (M+H)⁺

Das in der folgenden Tabelle aufgeführte Beispiel kann analog der für Beispiel XIII beschriebenen Vorschrift aus der Verbindung aus Beispiel X hergestellt werden.

| **Beispiel** | **Struktur** | **Analytische Daten** |
|---|---|---|
| **XIV** | 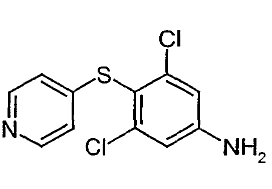 | ¹H-NMR (200 MHz, DMSO-d₆): δ = 6.32 (s, 2H), 6.83 (s, 2H), 6.90 (dd, 2H), 8.33 (dd, 2H). |
| | | HPLC (Methode 1): Rₜ = 3.80 min |
| | | MS (ESIpos): m/z = 270.9 (M+H)⁺ |

### Beispiel XV

### 3-Fluor-N-methyl-4-(4-pyridinylsulfanyl)anilin

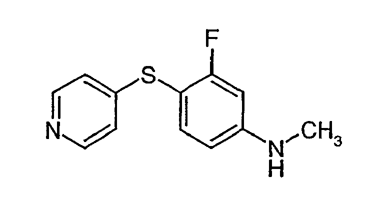

440.5 mg (2 mmol) der Verbindung aus Beispiel XI werden in 2 ml Methanol gelöst und mit 2 ml einer 21 %igen Natriumethanolatlösung versetzt. Es werden 84 mg (2.8 mmol) Paraformaldehyd zugegeben und über Nacht bei Raumtemperatur gerührt. Das Reaktionsgemisch wird mit 75.7 mg (2 mmol) Natriumborhydrid versetzt und 1.5 Stunden unter Rückfluss erhitzt. Nach dem Abkühlen wird mit 1M Kaliumhydroxidlösung vorsichtig hydrolysiert. Nachdem keine Reaktion mehr zu erkennen ist, wird der anfallende Feststoff abgesaugt und mit viel Wasser nachgewaschen. Der feste Rückstand wird in Diethylether gelöst, über Natriumsulfat getrocknet und im Vakuum einrotiert.

Es werden 387 mg (83 % d. Th.) Produkt erhalten.

¹H-NMR (200 MHz, DMSO-d₆): δ = 2.73 (d, 3H), 6.45-6.52 (m, 2H), 6.59-6.69 (m, 1H), 6.92 (dd, 2H), 7.29 (t, 1H), 8.32 (dd, 2H)
LC-MS (Methode 4): Rₜ = 2.35 min
MS (ESIpos): m/z = 235 (M+H)⁺

### Beispiel XVI

### N-(2-Amino-6-chlor-4-pyrimidinyl)-N-[3-fluor-4-(4-pyridinylsulfanyl)phenyl]amin

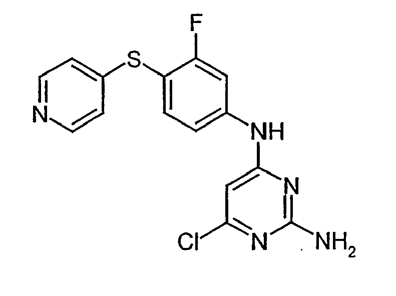

2.98 g (18.16 mmol) 2-Amino-4,6-dichlorpyrimidin werden in 300 ml Wasser suspendiert und dann mit 4 g (18.16 mmol) der Verbindung aus Beispiel XI und 1.82 ml konzentrierter Salzsäure versetzt. Über Nacht wird bei 100°C gerührt. Zur Aufarbeitung lässt man die Reaktionslösung abkühlen und stellt mit gesättigter Natriumhydrogencarbonatlösung basisch. Das dabei ausgefallene Produkt wird abgesaugt und im Vakuumtrockenschrank bei 40°C getrocknet.

Es werden 6.04 g (74 % d. Th.) Produkt erhalten.

¹H-NMR (200 MHz, DMSO-d₆): δ = 6.09 (s, 1H), 6.99 (dd, 4H), 7.41 (dd, 1H), 7.56 (dd, 1H), 8.27 (dd, 1H), 8.36 (dd, 2H), 9.89 (s, 1H)
HPLC (Methode 1): Rₜ = 3.69 min.
MS (ESIpos): m/z = 348 (M+H)⁺

Die in der folgenden Tabelle aufgeführten Beispiele können analog der oben für Beispiel XVI beschriebenen Vorschrift aus den entsprechenden Ausgangsverbindungen hergestellt werden.

| **Beispiel** | **Struktur** | **Analytische Daten** |
|---|---|---|
| **XVII** | 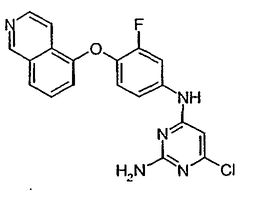 | LC-MS (Methode 3): Rₜ = 1.95 min |
| | | MS (ESIpos): m/z = 382 (M+H)⁺ |
| **XVIII** | 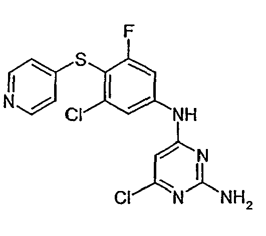 | ¹H-NMR (200 MHz, DMSO-d₆): δ = 6.08 (s, 1H), 7.00 (d, 2H), 7.09 (s, 2H), 7.74 (s, 1H), 8.15 (dd, 1H), 8.37 (dd, 2H), 10.00 (s, 1H) |
| | | HPLC (Methode 1): Rₜ = 3.81 min |
| | | MS (ESIpos): m/z = 382 (M+H)⁺ |
| **XIX** | 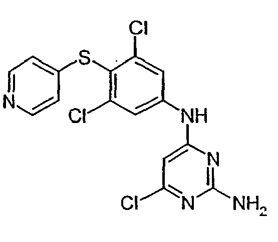 | ¹H-NMR (200 MHz, DMSO-d₆): δ = 4.80 (br.s, 1H), 6.34 (s, 1H), 7.69 (d, 2H), 8.36 (s, 2H), 8.71 (d, 2H), 10.72 (s, 1H) |
| | | HPLC (Methode 1): Rₜ = 3.8 min |
| | | MS (ESIpos): m/z = 398 (M+H)⁺ |

### Beispiel XX

### 5-Benzyl-1-oxa-5-azaspiro[4.2]heptan

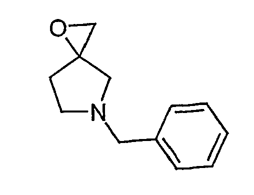

Die Verbindung kann nach einer Vorschrift von E.J. Corey et al. gemäß US 4,508,724 aus N-Benzyl-3-pyrrolidinon hergestellt.

### Beispiel XXI

### 1-Benzyl-3-hydroxy-3-(2-hydroxyethylaminomethyl)-pyrrolidin

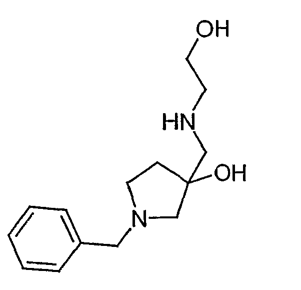

Man tropft 32.7 g (0.17 mol) von Beispiel XX zu 31 g (0.52 mol) Ethanolamin in 250 ml Wasser und rührt über Nacht bei Raumtemperatur. Man extrahiert mit Diethylether, engt die wässrige Phase ein und destilliert den Rückstand im Hochvakuum.

Es werden 42.1g (96 % d. Th.) Produkt erhalten.
Siedepunkt: 180 - 190°C/ 0.1 mbar

### Beispiel XXII

### 7-Benzyl-1-oxa-4,7-diazaspiro[5.4]dekan

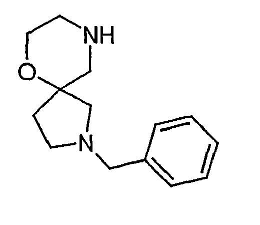

Man löst 85 g (340 mmol) von Beispiel XXI in einem Gemisch von 280 ml konzentrierter Schwefelsäure und 140 ml Wasser und erhitzt über Nacht auf 180°C. Man stellt mit 45%iger Natronlauge alkalisch, löst ausgeschiedene Salze mit Wasser und extrahiert fünfmal mit je 200 ml Chloroform. Man trocknet die organischen Phasen über Kaliumcarbonat, trennt das Trockenmittel ab und engt die Lösung ein. Der Rückstand wird im Hochvakuum destilliert.

Es werden 60 g (76 % d.Th.) des Produktes erhalten.
Siedepunkt: 125°C/0.08 mbar

### Beispiel XXIII

### 7-Benzyl-1-oxa-4,7-diazaspiro[5.4]dekan-4-carbonsäure-tert.-butylester

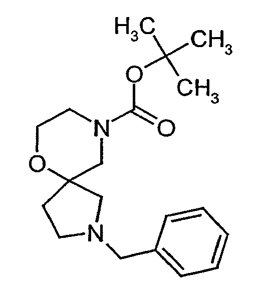

Zu 10.3 g (47 mmol) Beispiel XXII in 30 ml tert.-Butanol gibt man 2 g Natriumhydroxidplätzchen in 25 ml Wasser und tropft 11 g (50 mmol) Pyrokohlensäuredi-tert.-butylester hinzu. Man rührt über Nacht bei Raumtemperatur, versetzt mit 50 ml Wasser, extrahiert dreimal mit Chloroform, trocknet über Kaliumcarbonat, saugt das Trockenmittel ab, engt das Filtrat ein und destilliert den Rückstand im Hochvakuum.

Es werden 13.8 g (88 % d. Th.) des Produktes erhalten.
Siedepunkt: 160°C/ 0.3 mbar

### Beispiel XXIV

### 1-Oxa-4,7-diazaspiro[5.4]dekan-4-carbonsäure-tert.-butylester

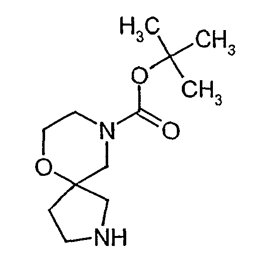

Man löst 13.7 g (41 mmol) von Beispiel XXIII, setzt 3 g 10 %ige Palladium-Aktivkohle hinzu und hydriert bei 100°C und 100 bar. Man saugt den Katalysator ab, engt das Filtrat ein und destilliert den Rückstand im Hochvakuum.

Es werden 7.6 g (75 % d.Th.) des Produktes erhalten.
Siedepunkt: 113°C/ 0.07 mbar

### Beispiel XXV

### 9-Methyl-6-oxa-2,9-diazaspiro[4.5]dekan

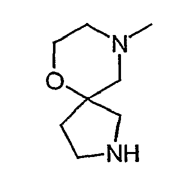

Die Verbindung kann aus Beispiel XXII durch reduktive Alkylierung nach der zur Herstellung von Beispiel XV beschriebenen Methode und anschließender hydrogenolytischer Abspaltung der Benzyl-Gruppe nach der Methode, die zur Herstellung von Beispiel XXIV beschrieben wurde, hergestellt werden.

### Beispiel XXVI

### 3,7-Dibenzyl-3,7-diazabicyclo[3.3.0]oktan-2,4-dion

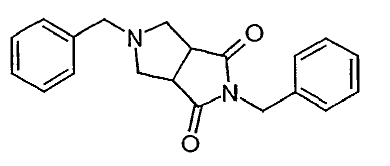

Man erhitzt 18.7 g (0.1 mol) N-Benzylimaleinimid mit 25 g (0.15 mol) N-Benzylglycin und 5 g (0.157 mol) Paraformaldehyd in 500 ml Toluol bis zum Ende der CO₂-Entwicklung unter Rückfluss. Man engt ein und verwendet das Rohprodukt ohne weitere Reinigung.

### Beispiel XXVII

### 3-Benzyl-3,7-diazabicyclo[3.3.0]oktan-2,4-dion

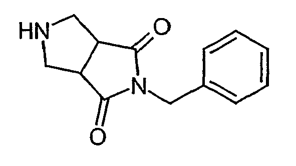

Man hydriert 150 g (0.4 mol) des Rohproduktes aus Beispiel XXVI in 750 ml Ethanol an 15 g 10 %iger Palladium-Aktivkohle bei 100°C und 100 bar. Man filtriert den Katalysator ab, engt die Lösung ein und filtriert mit Dichlormethan über 1.2 kg Kieselgel.

Es werden 38 g (42% d.Th.) des Produktes erhalten.

R_{f}-Wert: 0.35 (Laufmittel: Dichlormethan 96 % / Methanol 4 %).

### Beispiel XXVIII

### 3-Benzyl-3,7-diazabicyclo[3.3.0]oktan

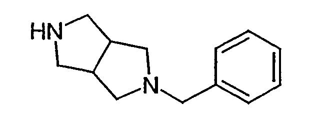

Man legt 16 g (0.42 mol) Lithiumaluminiumhydrid in 350 ml absolutem Tetrahydrofuran vor und tropft 38 g (0.165 mol) von Beispiel XXVII in 150 ml absolutem Tetrahydrofuran hinzu. Man erhitzt noch fünf Stunden unter Rückfluss, tropft je 16 ml Wasser, 16 %ige Kalilauge und wieder Wasser hinzu, saugt die anorganischen Salze ab und kocht sie zweimal mit Tetrahydrofuran aus. Die Filtrate werden eingeengt und im Membranpumpenvakuum destilliert.

Es werden 31.4 g (94 % d. Th.) des Produktes erhalten.
Siedepunkt: 140°C/4 mbar

### Beispiel XXIX

### 7-Benzyl-3,7-diazabicyclo[3.3.0]oktan-3-carbonsäure-tert.-butylester

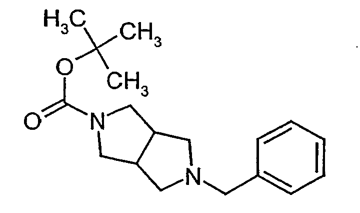

Man legt 6 g (29.7 mmol) Beispiel XXVIII vor, setzt 1.4 g (35 mmol) Natriumhydroxid in 30 ml Wasser hinzu und tropft dann 8 g (36.7 mmol) Pyrokohlensäure-tert.-butylester hinzu. Man rührt über Nacht bei Raumtemperatur, extrahiert mit Chloroform, trocknet über Kaliumcarbonat, filtriert das Trockenmittel ab, engt das Filtrat ein und destilliert den Rückstand.

Es werden 7.6 g (75 % d.Th.) des Produktes erhalten.
Siedepunkt: 153-156°C/0.2 mbar

### Beispiel XXX

### 3,7-Diazabicyclo[3.3.0]oktan-3-carbonsäure-tert.-butylester

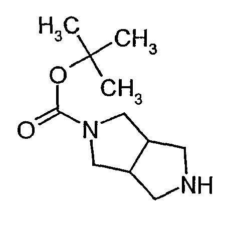

7.6 g (25.3 mmol) von Beispiel XXIX werden in 100 ml Ethanol an 1.5 g 10 %iger Palladium-Aktivkohle bei 100°C und 100 bar hydriert. Man filtriert den Katalysator ab, engt das Filtrat ein und destilliert den Rückstand.

Es werden 3.6 g (76 % d. Th.) des Produktes erhalten.
Siedepunkt: 92°C/0.08 mbar

### Beispiel XXXI

### 3,7-Diazabicyclo[3.3.0]oktan

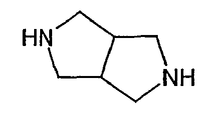

Die Verbindung kann aus Beispiel XXX durch Abspaltung der tert.-ButoxycarbonylGruppe mit Chlorwasserstoff (4 M in Dioxan) oder Trifluoressigsäure/Dichlormethan (1:1) hergestellt werden.

### Beispiel XXXII

### (4aR,7aS)-4-Benzyloktahydropyrrol[3,4-b][1,4]oxazin

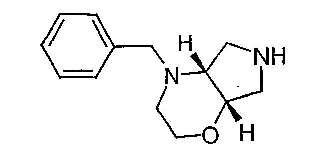

Die Herstellung der Verbindung ist in US 6 004 956 beschrieben.

### Beispiel XXXIII

### 5-Methyloktahydropyrrol[3,4-b]pyrrol

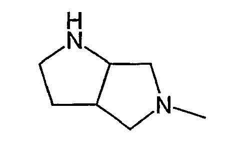

Die Herstellung der Verbindung ist in DE-A 4 032 560 beschrieben.

### Beispiel XXXIV

### [S,S]-8-Benzyl-2,8-diazabicyclo[4.3.0]nonan

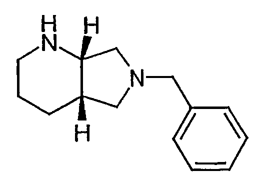

3 g (20 mmol) D(-)-Weinsäure werden in 10 ml Dimethylformamid durch Erwärmen auf 80°C gelöst und mit einer Lösung von 2.16 g (10 mmol) cis-8-Benzyl-2,8-diazabicyclo-[4.3.0]-nonan in 3 ml Dimethylformamid versetzt. Es wird 1 Stunde bei 0°C nachgerührt, dann wird abgesaugt und mit Dimethylformamid und Methoxyethanol gewaschen. (Ausbeute: 1.93 g)
Schmelzpunkt: 146-151°C
[α]_{D}²⁴= -19.3° (c= 1, H₂O)

Durch einmaliges Umkristallisieren aus Methoxyethanol wird diastereomerenreines [S,S]-8-Benzyl-2,8-diazabicyclo[4.3.0]nonan-D-tartrat erhalten.
Schmelzpunkt: 148-154°C
[α]_{D}²⁴= -22.7° (c= 1, H₂O)

40 g des Salzes werden in 250 ml Wasser gelöst und mit 32 g 45 %iger Natronlauge versetzt. Das ausgefallene Öl wird in 150 ml tert.-Butyl-methylether aufgenommen, die wässrige Phase wird nochmals mit 150 ml tert.-Butyl-methylether extrahiert. Die organischen Phasen werden vereinigt und nach dem Trocknen über Natriumsulfat eingeengt. Dann wird im Vakuum destilliert.

Es werden 18.5 g Produkt erhalten.
Siedepunkt: 107-109°C/0.1 mbar
[α]_{D}²⁴= 17.3° (unverdünnt)

### Beispiel XXXV

### [S,S]-2,8-Diazabicyclo[4.3.0]nonan

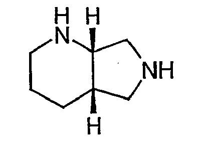

28.4 g (0.131 mol) [S,S]-8-Benzyl-2,8-diazabicyclo[4.3.0]nonan werden in 190 ml Methanol über 5.8 g Palladium auf Aktivkohle (5%) bei 90°C und 90 bar innerhalb von 5 Stunden hydriert. Dann wird der Katalysator abgesaugt, mit Methanol gewaschen und das Filtrat im Vakuum eingeengt. Der Rückstand wird ohne zu fraktionieren destilliert.

Es werden 15 g (91 % d. Th.) Produkt erhalten.
Siedepunkt: 44-59°C/ 0.18 mbar
[α]_{D}²⁴= -2.29° (unverdünnt)
ee > 99% (gaschromatographisch nach Derivatisierung mit Mosher's Reagenz bestimmt)

### Beispiel XXXVI

### [R,R]-8-Benzyl-2,8-diazabicyclo[4.3.0]nonan

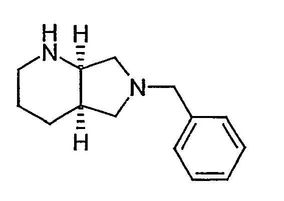

75 g (0.5 mol) L(+)-Weinsäure werden bei 80°C in 250 ml Dimethylformamid gelöst und 54.1 g (0.25 mol) cis-8-Benzyl-2,8-diazabicyclo[4.3.0]nonan als Lösung in 75 ml Dimethylformamid zugetropft. Es wird langsam auf 20°C abgekühlt und die Kristallsuspension 1 Stunde nachgerührt. Die Kristalle ([R,R]-8-Benzyl-2,8-diaza-bicyclo[4.3.0]nonan-L-tartrat) werden abgesaugt. (Das Filtrat kann weiter verarbeitet werden, um [S,S]-8-Benzyl-2,8-diazabicyclo[4.3.0]nonan zu erhalten). Diese Kristalle werden mit Dimethylformamid und Methoxyethanol gewaschen (Rohausbeute: 49.2 g) und aus 300 ml Methoxyethanol umkristallisiert. Man erhält 45.6 g enantiomerenreines [R,R]-8-Benzyl-2,8-diazabicyclo[4.3.0]nonan-L-tartrat (Enantiomerenreinheit gaschromatographisch nach Derivatisierung mit Chlorameisensäurementhylester bestimmt).
Schmelzpunkt: 121-124°C
[α]_{D}²⁴= +22.3° (c= 1, H₂O)

Das erhaltene Salz wird jetzt in die freie Base überführt. Dazu werden 44.5 g in 280 ml Wasser gelöst und mit 35.6 g 45 %iger Natronlauge versetzt. Das ausgefallene Öl wird in 170 ml tert.-Butyl-methylether aufgenommen, die wässrige Phase wird nochmals mit 170 ml tert.-Butyl-methylether extrahiert. Die organischen Phasen werden vereinigt und nach dem Trocknen über Natriumsulfat eingeengt. Dann wird im Vakuum destilliert.
Siedepunkt: 107-111°C/0.04 mbar
[α]_{D}²⁴= -17.5° (unverdünnt)

### Beispiel XXXVII

### [R,R]-2,8-Diazabicyclo[4.3.0]nonan

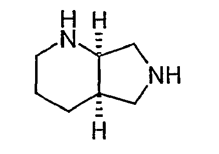

19.4 g (0.09 mol) [R,R]-8-Benzyl-2,8-diazabicyclo[4.3.0]nonan werden in 130 ml Methanol über 3.96 g Palladium auf Aktivkohle (5 %) bei 90°C und 90 bar innerhalb von 5 Stunden hydriert. Dann wird der Katalysator abgesaugt, mit Methanol gewaschen und das Filtrat im Vakuum eingeengt. Der Rückstand wird ohne zu fraktionieren destilliert.

Es werden 9.61 g (85 % d. Th.) Produkt erhalten.
Siedepunkt: 45-58°C/ 0.08 mbar
[α]_{D}²⁴= +2.30° (unverdünnt)

### Beispiel XXXVIII

### N-[2-Amino-6-(4-pyridinyl)-4-pyrimidinyl]-N-[3-fluor-4-(4-pyridinylsulfanyl)-phenyl] amin

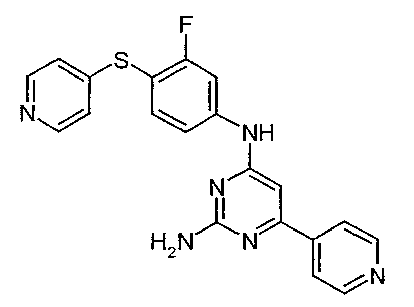

4.32 g (20.9 mmol) Beispiel V werden zusammen mit 4.61 g (20.9 mmol) Beispiel XI mit 29.6 ml Wasser versetzt. Die Mischung wird mit 9.25 ml konzentrierter Salzsäure versetzt und anschließend über Nacht bei 100°C gerührt. Nach Abkühlen auf Raumtemperatur wird mit Methanol verdünnt und mit gesättigter wässriger Natriumhydrogencarbonat-Lösung neutralisiert. Die Mischung wird auf Kieselgel aufgezogen und durch Säulenchromatographie an Kieselgel 60 mit Dichlormethan/Methanol 95:5 → Methanol gereinigt.
Ausbeute: 3.87 g (47 % d. Th.)
¹H-NMR (300 MHz, DMSO-d₆): δ = 6.65 (s, 1H), 6.71 (s, 2H), 7.02 (dd, 2H), 7.47-7.58 (m, 2H), 7.87 (dd, 2H), 8.30-8.41 (m, 3H), 8.72 (d, 2H), 9.90 (s, 1H)
LC-MS (Methode 1): Rₜ = 3.34 min
MS (ESIpos): m/z= 391 (M+H)⁺

### Beispiel XXXIX

### 3,4,5- Trifluornitrobenzol

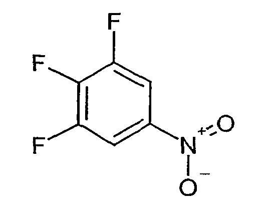

Die Verbindung ist zugänglich durch Oxidation von 3,4,5-Trifluoranilin mit Wasserstoffperoxid in Trifluoressigsäure gemäß einem Verfahren, das zur Herstellung analoger Derivate beschrieben ist in Heaton, A. et al., J. Fluorine Chem. 1997, 81 (2), 133-138 und Krapcho, A. P. et al., J. Org. Chem. 1990, 55 (21), 5662-5664.

Das in der folgenden Tabelle aufgeführte Beispiel kann analog der für Beispiel VII beschriebenen Vorschrift aus 4-Mercaptopyridin und 3,4,5-Trifluornitrobenzol hergestellt werden.

| **Beispiel** | **Struktur** | **Name** | **Analytische Daten** |
|---|---|---|---|
| **XL** | 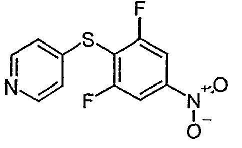 | 4-[(2,6-Difluor-4-nitrophenyl)sulfanyl]-pyridin | MS (ESI pos.): m/z = 269 (M+H)⁺ |

Das in der folgenden Tabelle aufgeführte Beispiel kann analog der für Beispiel XIII beschriebenen Vorschrift aus Beispiel XL hergestellt werden.

| **Beispiel** | **Struktur** | **Name** | **Analytische Daten** |
|---|---|---|---|
| **XLI** | 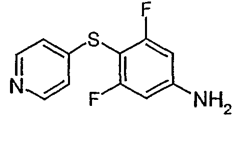 | 4-[(2,6-Difluor-4-aminophenyl)sulfanyl]pyridin | MS (ESI pos.): m/z = 239 (M+H)⁺, HPLC (Methode 1): Rₜ = 3.56 min |

Das in der folgenden Tabelle aufgeführte Beispiel kann analog der für Beispiel XVI beschriebenen Vorschrift aus den entsprechenden Ausgangsverbindungen hergestellt werden.

| **Beispiel** | **Struktur** | **Name** | **Analytische Daten** |
|---|---|---|---|
| **XLII** | 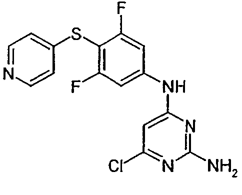 | N-(2-Amino-6-chlor-4-pyrimidinyl)-N-[3,5-difluor-4-(4-pyridinylsulfanyl)-phenyl]amin | MS (ESI pos.): m/z = 366 (M+H)⁺, HPLC (Methode 1): Rₜ= 3.75 min |

### Beispiel XLIII

### tert.-Butylhexahydropyrano[3,4-c]pyrrol-3a(4H)-ylmethylcarbamat

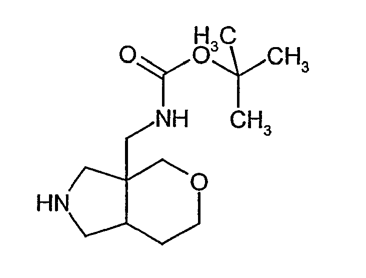

Die Verbindung kann nach einem in DE- A 4 032 560 beschriebenen Verfahren hergestellt werden.

### Beispiel XLIV

### tert.-Butylhexahydropyrrolo[3,4-b]pyrrole-5(1H)-carboxylat

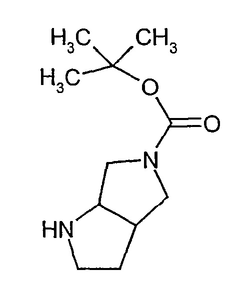

Die Verbindung kann nach einem in DE- A 4 032 560 beschriebenen Verfahren hergestellt werden.

### Beispiel XLV

### 2,3-Dihydro-1H-pyrrolo[3,4-c]pyridin

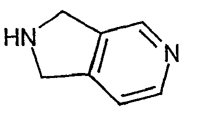

Die Verbindung kann nach einem in der Literatur beschriebenen Verfahren hergestellt werden: Gabriel Colman, Chem. Ber. 1902, 35, 845.

### Beispiel XLVI

### 4-(3-Pyrrolidinyl)morpholin

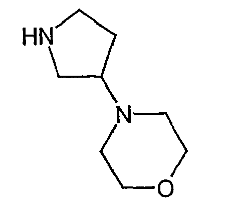

Die Verbindung kann nach einem in der Literatur beschriebenen Verfahren hergestellt werden: J. Org. Chem. 1998, 63 (23), 8266 - 8275.

### Ausführungsbeispiele

### Beispiel 1

### N-[2-Amino-6-(6-chinolinyl)-4-pyrimidinyl]-N-[3-fluor-4-(4-pyridinylsulfanyl)-phenyl]amin

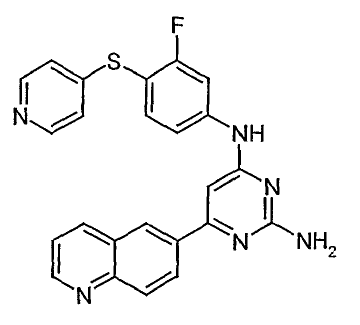

1.50 g (5.84 mmol) der Verbindung aus Beispiel II und 1.29 g (5.84 mmol) der Verbindung aus Beispiel XI werden zusammen in 70 ml Wasser vorgelegt und mit 10 Tropfen konzentrierter Salzsäure (37 %ig) versetzt. Die Suspension wird über Nacht bei 100°C gerührt. Nach dem Abkühlen wird das Reaktionsgemisch mit konzentrierter Natriumhydrogencarbonat-Lösung basisch gestellt und im Vakuum bis zur Trockene eingeengt.

Der Rückstand wird über eine präparative HPLC gereinigt und anschließend durch Flash-Chromatographie an Kieselgel mit Dichlormethan/Methanol 30/1 als Eluens gereinigt.

Es werden 330 mg (12 % d. Th.) Produkt erhalten.

¹H-NMR (300 MHz, DMSO-d₆): δ = 6.66 (br.s, 2H), 6.74 (s, 1H), 7.01 (d, 2H), 7.53 (d, 1H), 7.56-7.62 (m, 2H), 8.13 (d, 1H), 8.32-8.38 (m, 4H), 8.50 (d, 1H), 8.58 (d, 1H), 8.95 (dd, 1H), 9.87 (s, 1H)
HPLC (Methode 1): Rₜ = 4.22 min

Das in der folgenden Tabelle aufgeführte Beispiel kann analog der für Beispiel 1 beschriebenen Vorschrift aus den entsprechenden Ausgangsverbindungen hergestellt werden.

| **Beispiel** | **Struktur** | **Analytische Daten** |
|---|---|---|
| **2** | 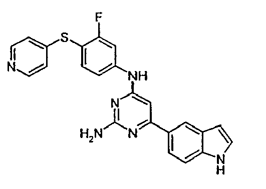 | ¹H-NMR (300 MHz, DMSO-d₆): δ = 6.44 (br.s, 2H), 6.54 (br.s, 1H), 6.60 (s, 1H), 6.99 (d, 1H), 7.01 (s, 1H), 7.40 (d, 1H), 7.45-7.54 (m, 4H), 7.74 (d, 1H), 8.23 (s, 1H), 8.36 (d, 3H), 9.68 (s, 1H) |
| | | HPLC (Methode 1): Rₜ=3.80 min |

### Beispiel 3

### N-[2-Amino-6-(6-chinolinyl)-4-pyrimidinyl]-N-[3-fluor-4-(5-isochinolinyloxy)-phenyl] amin

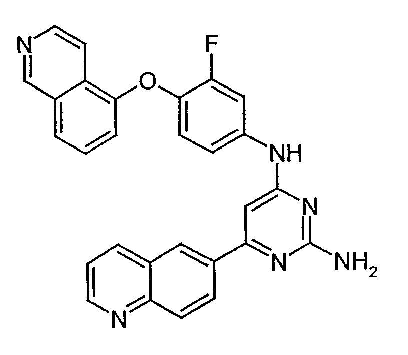

110 mg (0.29 mmol) der Verbindung aus Beispiel XVII werden in 12 ml eines Lösungsmittelgemisches aus Toluol und Ethanol im Verhältnis 2:1 suspendiert. 10 mg (0.01 mmol) Tetrakis(triphenylphosphin)palladium(0) werden zugegeben und 20 Minuten bei Raumtemperatur gerührt. Dann wird die Suspension mit 60 mg (0.35 mmol) 6-Chinolinboronsäure und 1 ml einer 2 M Natriumcarbonatlösung versetzt und über Nacht unter Rückfluss gerührt. Nach dem Abkühlen wird das Reaktionsgemisch im Vakuum eingeengt. Der Rückstand wird über Kieselgel 60 chromatographisch gereinigt (Laufmittel: Dichlormethan-Methanol 30:1 → 10:1).

Es werden 82 mg (60 % d. Th.) des Produktes erhalten.

¹H-NMR (200 MHz, DMSO-d₆): δ = 6.57 (br.s, 2H), 6.70 (s, 1H), 6.99 (d, 1H), 7.92 (t, 1H), 7.41-7.47 (m, 1H), 7.55-7.63 (m, 2H), 7.85 (d, 1H), 8.13 (t, 2H), 8.28-8.37 (m, 2H), 8.50 (br.d, 1H), 8.58-8.62 (m, 2H), 8.95 (dd, 1H), 9.39 (s, 1H), 9.66 (br.s, 1H)
HPLC (Methode 1): Rₜ= 3.49 min.
MS (ESIpos): m/z = 475 (M+H)⁺

Die in der folgenden Tabelle aufgeführten Beispiele können analog der für Beispiel 3 beschriebenen Vorschrift aus den entsprechenden Ausgangsverbindungen hergestellt werden.

| **Beispiel** | **Struktur** | **Analytische Daten** |
|---|---|---|
| **4** | 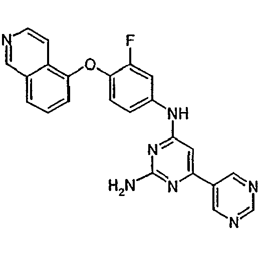 | ¹H-NMR (300 MHz, DMSO-d₆): δ = 6.84 (s, 1H), 7.01 (s, 1H), 7.18 (s, 1H), 7.30 (d, 1H), 7.41 (t, 1H), 7.58 (br.d, 1H), 7.80 (t, 1H), 8.12 (d, 1H), 8.29 (d, 1H), 8.44 (d, 1H), 8.70 (d, 1H) 9.30 (s, 2H), 9.41 (s, 1H), 9.75 (s, 1H), 11.25 (br.s, 1H) |
| | | LC-MS (Methode 5): Rₜ = 2.42 min |
| | | MS (ESIpos): m/z = 426 (M+H)⁺ |
| **5** | 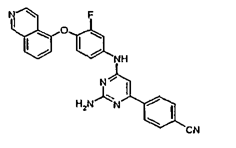 | LC-MS (Methode 5): Rₜ = 2.73 min |
| | | MS (ESIpos): m/z = 449 (M+H)⁺ |
| **6** | 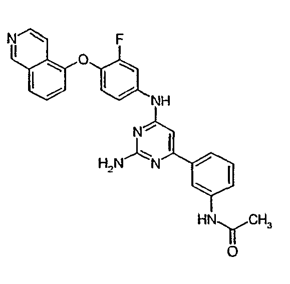 | ¹H-NMR (300 MHz, DMSO-d₆): δ = 2.11 (s, 3H), 6.68 (s, 1H), 7.25 (d, 1H), 7.40 (t, 1H), 7.53-7.66 (m, 4H), 7.67-7.76 (m, 1H), 7.76 (t, 1H), 8.08 (d, 1H), 8.28-8.39 (m, 4H), 8.68 (d, 1H), 9.68 (s, 1H), 10.34 (s, 1H), 11.21 (s, 1H) |
| | | LC-MS (Methode 5): Rₜ = 2.53 min |
| **7** | 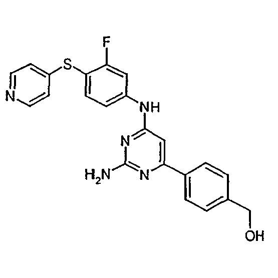 | ¹H-NMR (300 MHz, DMSO-d₆): δ = 4.62 (s, 2H), 6.86 (s, 1H), 7.46 (d, 2H), 7.58 (d, 2H), 7.69-7.77 (m, 2H), 7.88 (d, 2H), 8.41 (br.d, 1H), 8.56 (d, 2H), 11.51 (s, 1H) |
| | | LC-MS (Methode 3): Rₜ = 1.84 min |
| | | MS (ESIpos): m/z = 420 (M+H)⁺ |
| **8** | 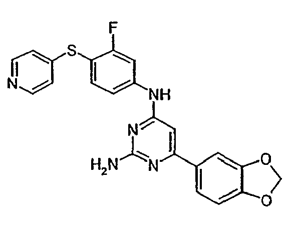 | ¹H-NMR (400 MHz, DMSO-d₆): δ = 6.20 (s, 2H), 6.89 (s, 1H), 7.20 (d, 1H), 7.48 (m, 2H), 7.55 (d, 2H), 7.74 (m, 2H), 8.43 (d, 1H), 8.60 (d, 2H), 11.69 (s, 1H) |
| | | LC-MS (Methode 3): Rₜ = 2.33 min |
| | | MS (ESIpos): m/z = 434 (M+H)⁺ |

### Beispiel 9

### N-{2-Amino-6-[3-(dimethylamino)-1-pyrrolidinyl]-4-pyrimidinyl}-N-[3-fluor-4-(4-pyridinyl-sulfanyl)phenyl]amin

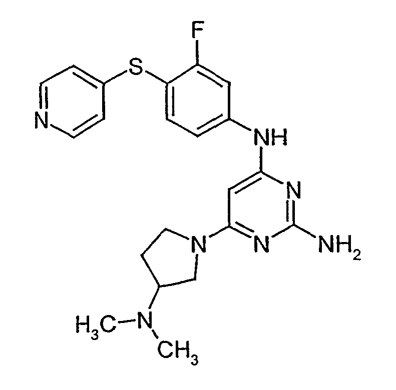

600 mg (1.73 mmol) der Verbindung aus Beispiel XVI werden in 40 ml Ethanol gelöst und mit 788 mg (6.9 mmol) 3-(Dimethylamino)-pyrrolidin und 3 ml (17.25 mmol) N,N-Diisopropylethylamin versetzt. Es wird über Nacht bei 80°C gerührt. Nach dem Abkühlen wird die Reaktionslösung über eine MPLC gereinigt (Laufmittel: Dichlormethan-Methanol 5:1 + 1% konzentrierter Ammoniaklösung).

Es werden 475 mg (58 % d. Th.) Produkt erhalten. (Enantiomerengemisch)

¹H-NMR (300 MHz, DMSO-d₆): δ = 1.69-1.83 (m, 1H), 2.06-2.15 (m, 1H), 2.20 (s, 6H), 2.69-2.80 (m, 1H), 3.05 (t, 1H), 3.43-3.70 (br.m, 2H), 4.05 (q, 1H), 5.16 (s, 1H), 5.95 (br.s, 2H), 6.97 (d, 2H), 7.36-7.47 (m, 2H), 8.21 (dd, 1H), 8.34 (dd, 2H), 9.23 (s, 1H)
LC-MS (Methode 7): Rₜ = 0.44 min
MS (ESIpos): m/z = 426 (M+H)⁺

Die folgenden beiden Enantiomere werden aus Beispiel 9 durch Enantiomerentrennung mittels chiraler HPLC (Methode 11) gewonnen.

| **Beispiel** | **Struktur** | **Analytische Daten** |
|---|---|---|
| **10** | 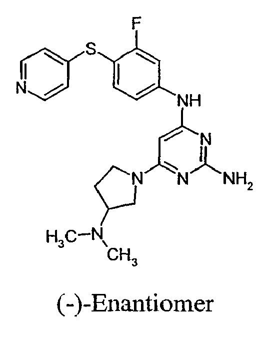 | Chirale HPLC (Methode 11): Rₜ = 6.79 min |
| **11** | 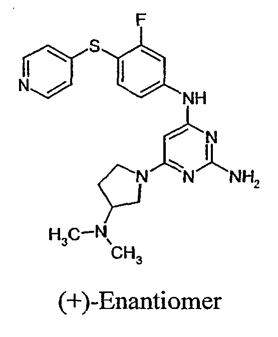 | Chirale HPLC (Methode 11): Rₜ = 5.77 min |

Die in der folgenden Tabelle aufgeführten Beispiele können analog der für Beispiel 9 beschriebenen Vorschrift aus den entsprechenden Ausgangsverbindungen hergestellt werden.

| **Beispiel** | **Struktur** | **Analytische Daten** |
|---|---|---|
| **12** | 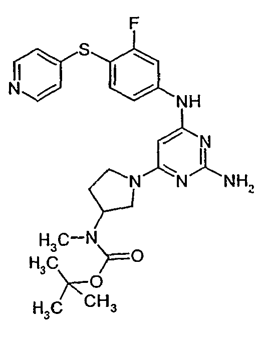 | ¹H-NMR (300 MHz, DMSO-d₆): δ = 1.42 (s, 9H), 2.07 (m, 2H), 2.74 (s, 3H), 3.20 (m, 2H), 3.52 (m, 2H), 4.63 (q, 1H), 5.17 (s, 1H), 6.03 (br.s, 2H), 6.97 (d, 2H), 7.33-7.49 (m, 2H), 8.25 (dd, 1H), 8.34 (d, 2H), 9.29 (s, 1H) |
| | | LC-MS (Methode 6): Rₜ = 1.79 min |
| | | MS (ESIpos): m/z = 512 (M+H)⁺ |
| **13** | 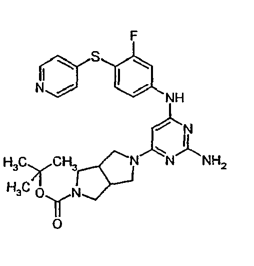 | ¹H-NMR (300 MHz, DMSO-d₆): δ = 1.39 (s, 9H), 2.92 (m, 2H), 3.16 (m, 4H), 3.52 (m, 4H), 5.16 (s, 1H), 5.95 (br.s, 2H), 6.97 (d, 2H), 7.36-7.47 (m, 2H), 8.20 (dd, 1H), 8.34 (d, 2H), 9.25 (s, 1H) |
| | | LC-MS (Methode 7): Rₜ = 3.04 min |
| | | MS (ESIpos): m/z = 524 (M+H)⁺ |
| **14** | 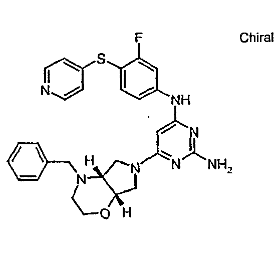 | ¹H-NMR (200 MHz, CDCl₃): δ = 1.57-1.83 (m, 1H), 2.43-2.56 (m, 1H), 2.68-2.99 (m, 1H), 3.30-3.52 (m, 3H), 3.57-3.98 (m, 6H), 4.14 (br.s, 1H), 4.66 (br.s, 1H), 5.12 (br,s, 1H), 5.30 (s, 1H), 6.52 (s, 1H), 6.94 (d, 1H), 7.07 (dd, 1H), 7.28-7.48 (m, 6H), 7.60 (dd, 1H), 8.35 (d, 2H) |
| | | LC-MS (Methode 7): Rₜ = 2.90 min |
| | | MS (ESIpos): m/z = 530 (M+H)⁺ |
| **15** | 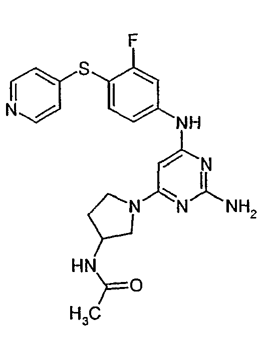 | ¹H-NMR (400 MHz, DMSO-d₆): δ = 1.70-1.90 (m, 2H), 1.78 (s, 3H), 2.68-3.01 (m, 1H), 3.16-3.61 (m, 3H), 4.18-4.32 (m, 1H), 5.15 (s, 1H), 5.98-6.10 (br.s, 2H), 6.98 (d, 2H), 7.31-7.49 (m, 2H), 8.14 (d, 1H), 8.28 (dd, 1H), 8.38 (dd, 2H), 9.29 (s, 1H) |
| | | LC-MS (Methode 7): Rₜ= 2.58 min |
| | | MS (ESIpos): m/z = 440 (M+H)⁺ |

### Beispiel 16

### N-[1-(2-Amino-6-{[3-fluor-4-(4-pyridinylsulfanyl)phenyl]amino}-4-pyrimidinyl)-3-pyrrolidinyl]-N-methylamin

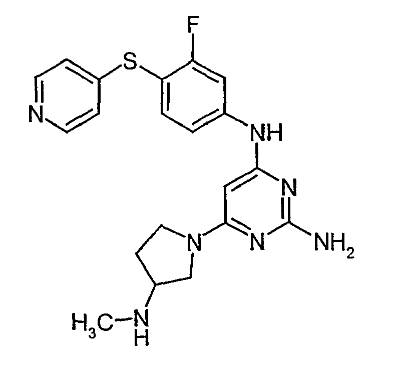

46 mg (0.09 mmol) der Verbindung aus Beispiel 12 werden mit 8 ml 4 M Salzsäure in Dioxan versetzt und 4 Stunden bei Raumtemperatur gerührt. Anschließend wird im Vakuum eingeengt. Der Rückstand wird mit wenig konzentrierter Ammoniaklösung versetzt, einrotiert und dann mit 2 ml Wasser aufgeschlämmt. Der Niederschlag wird abgesaugt und mit 2 ml Dichlormethan nachgewaschen.

Man erhält 23 mg (62 % d. Th.) Produkt.

¹H-NMR (300 MHz, DMSO-d₆): δ = 1.40 (s, 3H), 2.13-2.40 (m, 2H), 2.60 (s, 3H), 3.51-3.90 (m, 6H), 5.29 (s, 1H), 7.00 (d, 1H), 7.16 (s, 1H), 7.33-7.52 (m, 3H), 8.18 (br.d, 1H), 8.36 (d, 1H)
LC-MS (Methode 1): Rₜ = 0.68 min
MS (ESIpos): m/z = 412 (M+H)⁺

### Beispiel 17

### N-[2-Amino-6-(4-cyclopentyl-1-piperazinyl)-4-pyrimidinyl]-N-[3-fluor-4-(4-pyridinyl-sulfanyl)phenyl]amin

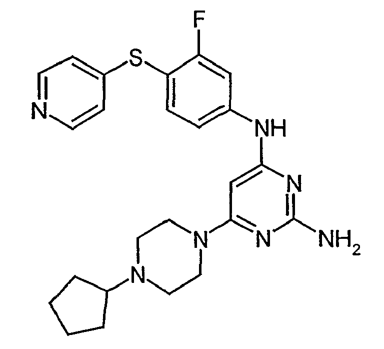

300 mg (0.86 mmol) der Verbindung aus Beispiel XVI werden in 8 ml 2-Ethyl-1-hexanol suspendiert und mit 266 mg (1.73 mmol) 1-Cyclopentylpiperazin und 0.75 ml (4.31 mmol) N,N-Diisopropylethylamin versetzt. Es wird über Nacht bei 150°C gerührt. Nach dem Abkühlen wird die Reaktionslösung über eine MPLC gereinigt (Laufmittel: Dichlormethan-Methanol 10:1 + 1% konzentrierter Ammoniaklösung).

Es werden 216 mg (52 % d. Th.) Produkt erhalten.

¹H-NMR (400 MHz, DMSO-d₆): δ = 1.28-1.41 (m, 2H), 1.44-1.55 (m, 2H), 1.57-1.68 (m, 2H), 1.70-1.85 (m, 2H), 2.43 (br.s, 5H), 3.41 (br.s, 4H), 5.39 (s, 1H), 6.04 br.s, 2H), 6.97 (d, 2H), 7.36 (dd, 1H), 7.45 (t, 1H), 8.23 (dd, 1H), 8.34 (d, 2H), 9.28 (s, 1H)
LC-MS (Methode 7): Rₜ = 2.38 min
MS (ESIpos): m/z = 466 (M+H)⁺

Die in der folgenden Tabelle aufgeführten Beispiele können analog der für Beispiel 17 beschriebenen Vorschrift aus den entsprechenden Ausgangsverbindungen hergestellt werden.

| **Beispiel** | **Struktur** | **Analytische Daten** |
|---|---|---|
| **18** | 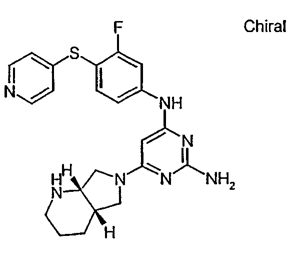 | ¹H-NMR (400 MHz, DMSO-d₆): δ = 1.21-1.74 (m, 5H), 2.09-2.33 (m, 1H), 2.72-2.93 (m, 2H), 3.06-3.26 (m, 3H), 3.42-3.65 (m, 1H), 5.12 (s, 1H), 5.76 (s, 1H), 5.95 (br.s, 2H), 6.98 (d, 2H), 7.33-7.49 (m, 2H), 8.24 (dd, 1H), 8.34 (d, 2H), 9.25 (s, 1H) |
| | | LC-MS (Methode 6): Rₜ = 0.34 min |
| | | MS (ESIpos): m/z = 438 (M+H)⁺ |
| **19** | 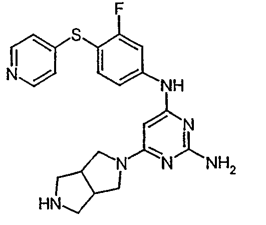 | LC-MS (Methode 7): Rₜ = 0.41 min |
| | | MS (EIneg): m/z = 422 (M-) |
| **20** | 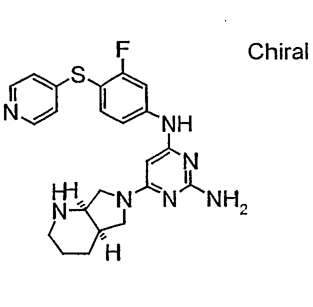 | ¹H-NMR (200 MHz, DMSO-d₆): δ 1.23-1.75 (m, 5H), 2.21 (br.s, 2H), 2.84 (d, 1H), 3.22-3.63 (m, 4H), 4.11 (q, 1H), 5.11 (s, 1H), 5.94 (br.s, 2H), 6.97 (d, 2H), 7.32-7.49 (m, 2H), 8.29 (dd, 1H), 8.34 (d, 2H), 9.23 (s, 1H) |
| | | LC-MS (Methode 7): Rₜ = 0.37 min |
| | | MS (ESIpos): m/z = 438 (M+H)⁺ |
| **22** | 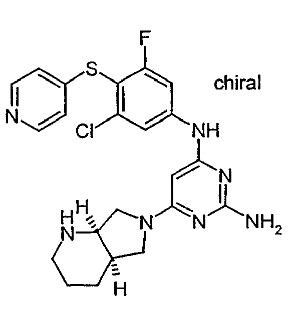 | ¹H-NMR (300 MHz, DMSO-d₆): δ = 1.35-1.72 (m, 5H), 2.22-2.38 (m, 1H), 2.57 (t, 1H), 2.84-2.93 (m, 1H), 3.22-3.63 (m, 4H), 4.06 (br.s, 1H), 5.14 (s, 1H), 6.00 (s, 2H), 6.97 (d, 2H), 7.74 (s, 1H), 8.12 (dd, 1H), 8.36 (d, 2H), 9.42 (s, 1H) |
| | | MS (ESIpos): m/z = 472 (M+H)⁺ |
| | | LC/MS (Methode 1): Rₜ = 3.53 min |
| **23** | 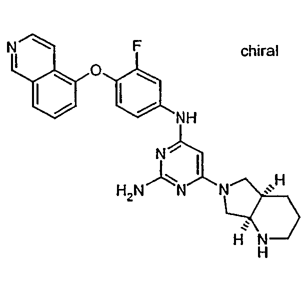 | ¹H-NMR (400 MHz, DMSO-d₆): δ = 1.34-1.66 (m, 5H), 2.04-2.29 (m, 2H), 2.80-2.83 (m, 1H), 3.17 (d, 3H), 4.09 (q, 1H), 5.07 (s, 1H), 5.75 (s, 1H), 5.82 (s, 2H), 6.93 (d, 1H), 7.12-7.34 (m, 2H), 7.56 (t, 1H), 7.82 (d, 1H), 8.10 (d, 1H), 8.17 (dd, 1H), 8.59 (d, 1H), 8.92 (s, 1H), 9.36 (s, 1H) |
| | | MS (ESIpos): m/z = 472 (M+H)⁺ |
| **24** | 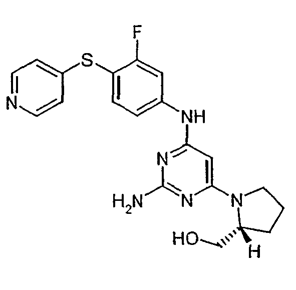 | ¹H-NMR (400 MHz, DMSO-d₆): δ = 1.82-2.10 (m, 5H), 3.29-3.30 (m, 4H), 3.41-3.48 (m, 1H), 3.54-3.65 (m, 2H), 4.15 (br.s, 1H), 5.29 (s, 1H), 7.02 (d, 2H), 7.34 (dd, 1H), 7.42 (t, 1H), 7.93 (dd, 1H), 8.25 (d, 2H) |
| | | MS (ESIpos): m/z = 413 (M+H)⁺ |
| | | HPLC (Methode 1): Rₜ = 3.48 min |
| **25** | 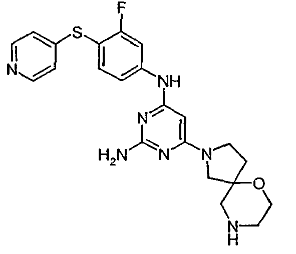 | MS (CIpos): m/z = 454 (M+H)⁺ |
| | | HPLC (Methode 1): Rₜ = 3.39 min |
| | aus Beispiel XVI und Beispiel XXIV; bei der chromatographischen Reinigung unter Säurezusatz wird die Boc-Gruppe abgespalten | |
| **26** | 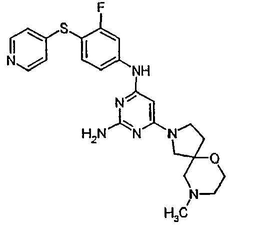 | MS (ESIpos): m/z = 468 (M+H)⁺ |
| | | HPLC (Methode 1): Rₜ = 3.41 min |
| **27** | 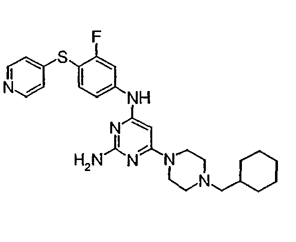 | ¹H-NMR (300 MHz, DMSO-d₆):δ =0.92-1.03 (m, 2H), 1.13-1.30 (m, 5H), 1.64-1.80 (m, 6H), 2.99 (d, 6H), 4.22 (d, 2H), 5.53 (s, 1H), 6.42 (br.s, 1H), 7.22 (d, 2H), 7.40 (dd, 1H), 7.52 (t, 1H), 8.19 (d, 1H), 8.45 (d, 2H), 9.31 (br.s, 1H), 9.57 (s, 1H) |
| | | MS (ESIpos): m/z = 494 (M+H)⁺ |
| | | HPLC (Methode 1): Rₜ = 3.77 min |
| **28** | 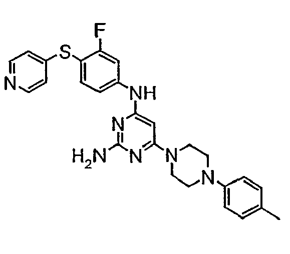 | ¹H-NMR (300 MHz, DMSO-d₆): δ = 2.50 (s, 3H), 3.20 (br.s, 4H), 3.64 (br.s, 4H), 5.61 (s, 1H), 6.89 (d, 3H), 7.06 (d, 3H), 7.19 (d, 2H), 7.38 (dd, 1H), 7.5 (t, 1H), 8.07 (br.s, 1H), 8.44 (d, 2H), 9.85 (br.s, 1H) |
| | | MS (ESIpos): m/z = 488 (M+H)⁺ |
| | | HPLC (Methode 1): Rₜ= 3.81 min |
| **29** | 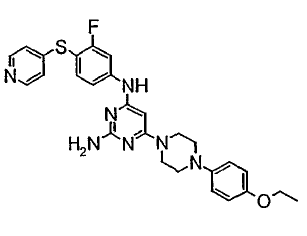 | ¹H-NMR (400 MHz, MeOH-d₄): δ = 1.35 (t, 3H), 3.19 (t, 4H), 3.75 (s, 4H), 3.98 (q, 2H), 4.89 (s, 1H), 6.85 (d, 2H), 6.99 (d, 2H), 7.43 (d, 3H), 7.65 (t, 1H), 7.84 (br.s, 1H), 8.43 (d, 2H) |
| | | MS (ESIpos): m/z = 518 (M+H)⁺ |
| | | HPLC (Methode 1): Rₜ = 3.82 min |
| **30** | 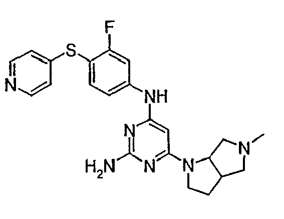 | ¹H-NMR (400 MHz, MeOH-d₄): δ = 1.29-1.41 (m, 2H), 2.03 (br.s, 1H), 2.32 (br.s, 1H), 2.67 (s, 2H), 2.95 (s, 5H), 3.61-3.68 (m, 2H), 4.67 (s, 1H), 7.14 (d, 3H), 7.38 (dd, 1H), 7.52 (t, 2H), 7.93 (d, 1H), 8.32 (dd, 3H) |
| | | HPLC (Methode 1): Rₜ = 3.35 min |
| **31** | 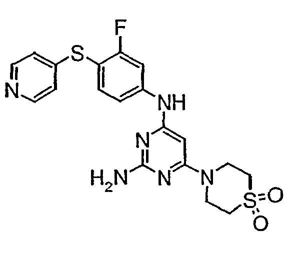 | ¹H-NMR (200 MHz, DMSO-d₆): δ = 3.17 (br.s, 4H), 3.97 (br.s, 4H), 5.61 (s, 1H), 7.29 (d, 3H), 7.39 (dd, 1H), 7.54 (t, 1H), 8.15 (br.d, 1H), 8.48 (d, 3H), 9.61 (br.s, 1H) |
| | | MS (ESIpos): m/z = 447 (M+H)⁺ |
| | | HPLC (Methode 1): Rₜ = 3.38 min |
| **32** | 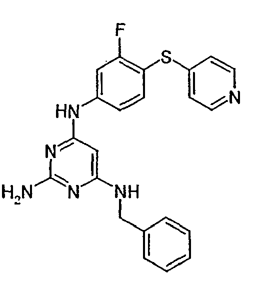 | ¹H-NMR (200 MHz, DMSO-d₆): δ = 4.47 (d, 3H), 5.41 (s, 1H), 7.21 (d, 3H), 7.32 (d, 4H), 7.43-7.48 (m, 1H), 7.61 (t, 1H), 7.64-7.86 (m, 1H), 8.00-8.16 (m, 1H), 8.24-8.33 (m, 1H), 8.46 (d, 2H), 10.17 (s, 1H) |
| | | MS (ESIpos): m/z = 419 (M+H)⁺ |
| | | HPLC (Methode 1): Rₜ= 3.85 min |
| **33** | 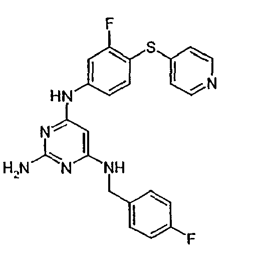 | ¹H-NMR (200 MHz, DMSO-d₆): δ = 4.43-4.50 (m, 3H), 5.39 (s, 1H), 7.15-7.29 (m, 5H), 7.32-7.44 (m, 4H), 7.52-7.66 (m, 2H), 8.44 (dd, 2H), 10.14 (br.s, 1H) |
| | | MS (ESIpos): m/z = 437 (M+H)⁺ |
| | | HPLC (Methode 1): Rₜ = 3.89 min |
| **34** | 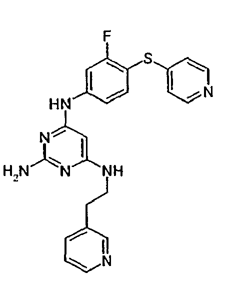 | ¹H-NMR (200 MHz, DMSO-d₆): δ = 1.22 (d, 1H), 1.26 (d, 1H), 3.52 (m, 2H), 5.51 (s, 1H), 7.38 (d, 3H), 7.64 (t, 2H), 7.72-7.78 (m, 2H), 8.19 (d, 2H), 8.53 (d, 2H), 8.66-8.73 (m, 3H), 10.26 (s, 1H) |
| | | MS (ESIpos): m/z = 434 (M+H)⁺ |
| | | HPLC (Methode 1): Rₜ = 3.34 min |
| **35** | 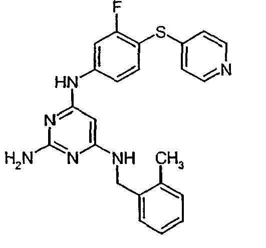 | MS (ESIpos): m/z = 433 (M+H)⁺ |
| | | LC/MS (Methode 4): Rₜ= 2.90 min |
| **36** | 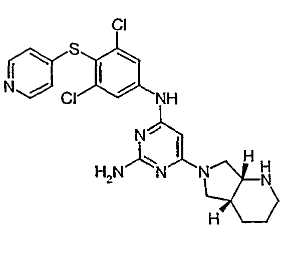 | ¹H-NMR (200 MHz, DMSO-d₆): δ = 1.04-1.10 (m, 1H), 1.44-1.66 (m, 6H), 2.47-2.62 (m, 1H), 2.69-2.84 (m, 2H), 2.96-3.13 (m, 4H), 5.06 (s, 1H), 6.88 (d, 2H), 7.91 (s, 2H), 8.23 (d, 2H), 8.83 (br.s, 1H), 9.55 (br.s, 1H) |
| | | MS (ESIpos): m/z = 488 (M+H)⁺ |
| | | HPLC (Methode 1): Rₜ = 3.61 min |
| **37** | 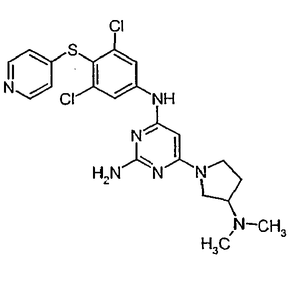 | ¹H-NMR (400 MHz, DMSO-d₆): δ = 1.22-1.28 (m, 1H), 2.15-2.26 (m, 1H), 2.84 (s, 6H), 3.17 (s, 1H), 3.82 (m, 3H), 3.92-4.02 (m, 2H) 5.26 (s, 1H), 7.07 (d, 2H), 8.09 (s, 2H), 8.42 (d, 2H), 9.71 (br.s, 1H), 10.05 (br.s, 1H) |
| | | HPLC (Methode 1): Rₜ = 3.57 min |
| **38** | 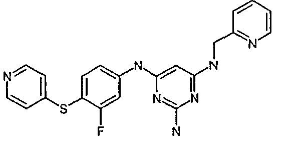 | MS (ESIpos): m/z = 419 (M+H)⁺ |
| | | LC-MS (Methode 4): Rₜ = 2.30 min |

### Beispiel 39

### N-(2-Amino-4,5'-bipyrimidin-6-yl)-N-[3-fluor-4-(4-pyridinylsulfanyl)phenyl]amin

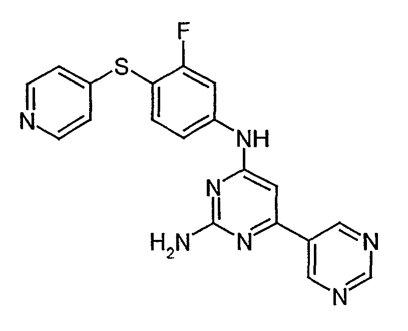

100 mg (0.253 mmol) der Verbindung Beispiel XI werden in 10 ml Dimethylformamid vorgelegt und mit 350 mg (2.53 mmol) Kaliumcarbonat versetzt. Es werden 52.13 mg (2.53 mmol) 5-(4,4,5,5-Tetramethyl-1,3,2-dioxaborolan-2-yl)pyrimidin und 20.7 mg (0.025 mmol) 1,1'-Bis(diphenylphosphin)ferrocen-dichlorpalladium(II)-komplex mit Dichlormethan im Argongegenstrom zugegeben. Die Reaktionslösung färbt sich nach kurzer Zeit schwarz. Es wird über Nacht bei 120°C gerührt. Nach dem Abkühlen wird das Reaktionsgemisch mit ca. 30 ml Ethylacetat verdünnt und mit Wasser extrahiert. Die wässrige Phase wird noch zweimal mit 20 ml Ethylacetat extrahiert. Die organische Phase wird einmal mit gesättigter Natriumchloridlösung gewaschen, über Natriumsulfat getrocknet und im Vakuum eingeengt. Der Rückstand wird über Kieselgel 60 (Laufmittel: Dichlormethan-Methanol 20:1) und anschließend über eine präparative HPLC gereinigt.

Man erhält 9 mg (8% d. Th.) des Produktes.

¹H-NMR (400 MHz, DMSO-d₆): δ = 6.66 (s, 1H), 7.33 (d, 2H), 7.55-7.65 (m, 2H), 8.40 (dd, 1H), 8.50 (br.s, 2H), 9.25 (s, 2H), 9.32 (s, 1H), 10.26 (br.s, 1H)
MS (ESIpos): m/z = 392 (M+H)⁺
HPLC (Methode 1): Rₜ = 3.53 min

### Beispiel 40

### N-{2-Amino-6-[4-(2-pyridinyl)-1-piperazinyl]-4-pyrimidinyl}-N-[3-fluor-4-(4-pyridinyl-sulfanyl)phenyl]amin

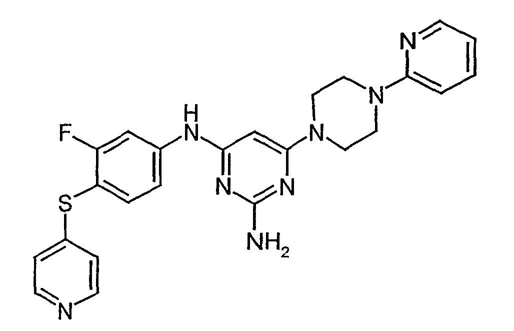

34.78 mg (0.1 mmol) der Verbindung aus Beispiel XVI werden in 0.5 ml Dimethylformamid gelöst und mit 32.6 mg (0.2 mmol) 1-(2-Pyridinyl)piperazin und 25.8 mg (0.2 mmol) N,N-Diisopropylethylamin versetzt. Es wird über Nacht bei 120°C gerührt. Zur Aufarbeitung wird die Reaktionslösung zuerst über eine LCMS gereinigt, anschließend noch einmal über eine UV/HPLC.
Es werden 13.1 mg (20 % d. Th.) Produkt erhalten.
LC-MS (Methode 4): Rₜ = 1.30 min
MS (ESIpos): m/z = 475 (M+H)⁺

Die in der folgenden Tabelle aufgeführten Beispiele können analog der für Beispiel 40 beschriebenen Vorschrift aus Beispiel XVI und den entsprechenden Ausgangsverbindungen hergestellt werden.

| **Beispiel** | **Struktur** | **Analytische Daten** |
|---|---|---|
| **41** | 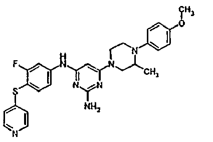 | LC-MS (Methode 4): Rₜ = 2.80 min MS (ESIpos): m/z = 518 (M+H)⁺ |
| **42** | 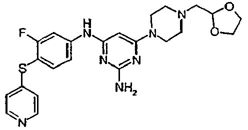 | LC-MS (Methode 4): Rₜ = 0.70 min MS (ESIpos): m/z = 484 (M+H)⁺ |
| **43** | 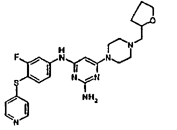 | LC-MS (Methode 4): Rₜ = 0.70 min MS (ESIpos): m/z = 482 (M+H)⁺ |
| **44** | 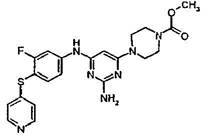 | LC-MS (Methode 4): Rₜ = 2.40 min MS (ESIpos): m/z = 456 (M+H)⁺ |
| **45** | 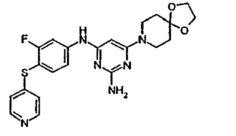 | LC-MS (Methode 4): Rₜ = 2.40 min MS (ESIpos): m/z = 455 (M+H)⁺ |
| **46** | 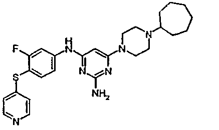 | LC-MS (Methode 4): Rₜ = 2.20 min MS (ESIpos): m/z = 494 (M+H)⁺ |
| **47** | 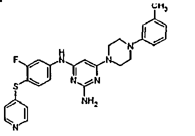 | LC-MS (Methode 4): Rₜ = 3.10 min MS (ESIpos): m/z = 4.88 (M+H)⁺ |
| **48** | 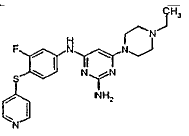 | LC-MS (Methode 4): Rₜ = 0.50 min MS (ESIpos): m/z = 426 (M+H)⁺ |
| **49** | 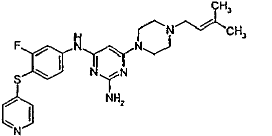 | LC-MS (Methode 4): Rₜ = 1.30 min MS (ESIpos): m/z = 466 (M+H)⁺ |
| **50** | 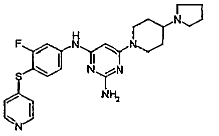 | LC-MS (Methode 4): Rₜ = 0.50 min MS (ESIpos): m/z = 466 (M+H)⁺ |
| **51** | 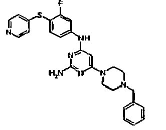 | LC-MS (Methode 4): Rₜ = 1.90 min MS (ESIpos): m/z = 488 (M+H)⁺ |
| **52** | 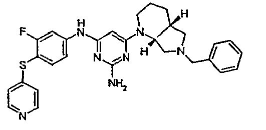 | LC-MS (Methode 4): Rₜ = 2.30 min MS (ESIpos): m/z = 528 (M+H)⁺ |

### Beispiel 53

### N-[2-Amino-6-(4-methylphenoxy)-4-pyrimidinyl]-N-[3-fluor-4-(4-pyridinylsulfanyl) phenyl]amin

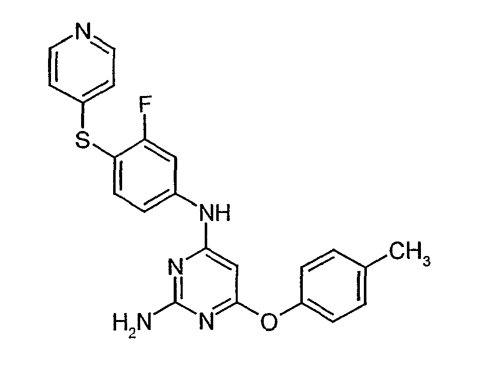

30 mg (0.086 mmol) der Verbindung aus Beispiel XVI werden mit 37.31 mg (0.345 mmol) p-Kresol und 9.679 mg (0.173 mmol) festem Kaliumhydroxid gut verengt und unter Argon als Schmelze für 3 Stunden bei 150°C gerührt. Zur Aufarbeitung wird die Schmelze über eine Säulenchromatographie an Kieselgel 60 mit Dichlormethan/ Methanol 95:5 gereinigt.

Es werden 15 mg (41 % d. Th.) Produkt erhalten.

LC-MS (Methode ): Rₜ = 2.40 min
MS (ESIpos): m/z = 420 (M+H)⁺

Die in der folgenden Tabelle aufgeführten Beispiele können analog der für Beispiel 53 beschriebenen Vorschrift aus den entsprechenden Ausgangsverbindungen hergestellt werden.

| **Beispiel** | **Struktur** | **Analytische Daten** |
|---|---|---|
| **54** | 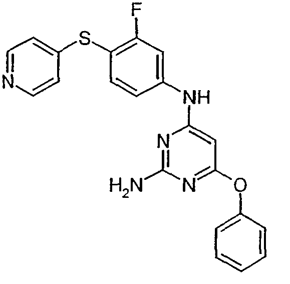 | LC-MS (Methode 10): Rₜ = 2.20 min MS (ESIpos): m/z = 406 (M+11)⁺ |
| **55** | 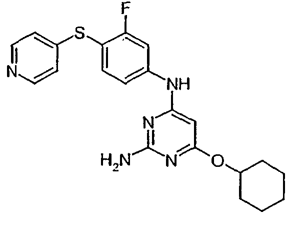 | LC/MS (Methode 10): Rₜ = 2.27 min MS(ESIpos): m/z = 412 (M+H)⁺ |
| **56** | 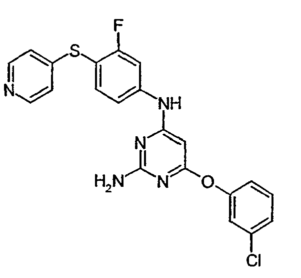 | LC/MS (Methode 10): Rₜ =2.56 min MS(ESIpos): m/z = 440 (M+H)⁺ |

Die in der folgenden Tabelle aufgeführten Beispiele können analog der für Beispiel 17 beschriebenen Vorschrift aus den entsprechenden Ausgangsverbindungen hergestellt werden.

| **Beispiel** | **Struktur** | **Analytische Daten** |
|---|---|---|
| **57** | 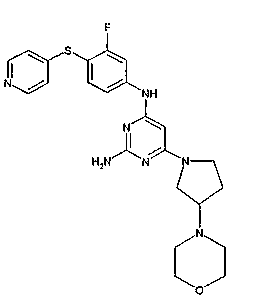 | HPLC (Methode 1): Rₜ = 3.20 min MS (ESIpos): m/z = 468 (M+H)⁺ |
| **58** | 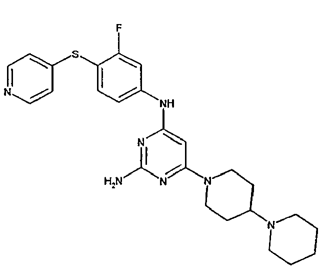 | HPLC (Methode 1): Rₜ = 3.30 min MS (ESIpos): m/z = 480 (M+H)⁺ |
| **59** | 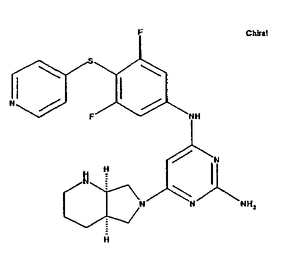 | HPLC (Methode 1): Rₜ = 3.42 min MS (ESIpos): *m*/*z* = 456 (M+H)⁺ MS (ESIneg): m/z = 454 (M+H)⁻ |
| **60** | 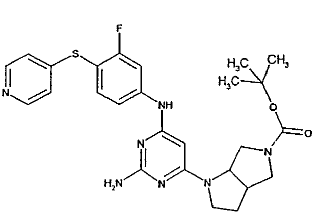 | HPLC (Methode 1): Rₜ = 3.90 min MS (ESIpos): m/z = 524 (M+H)⁺ |
| **61** | 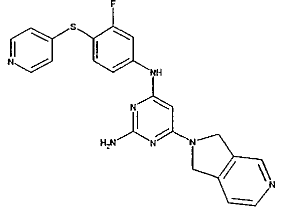 | LC-MS (Methode 5): Rₜ = 0.85 min HPLC (Methode 1): Rₜ = 3.30 min |
| **62** | 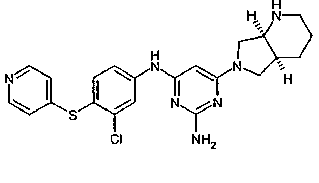 | LC-MS (Methode 13): Rₜ = 1.77 min MS (ESIpos): m/z = 454 (M+H)⁺ |
| **63** | 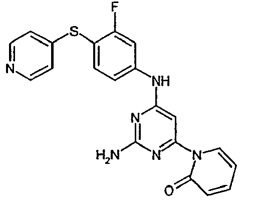 | LC-MS (Methode 13): Rₜ = 2.63 min MS (ESIpos): m/z = 407 (M+H)⁺ |
| **64** | 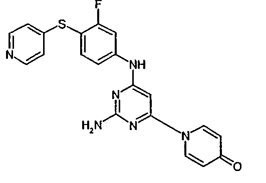 | LC-MS (Methode 15): Rₜ = 1.59 min MS (ESIpos): m/z = 407 (M+H)⁺ |

Die in der folgenden Tabelle aufgeführten Beispiele können analog der für Beispiel 53 beschriebenen Vorschrift aus den entsprechenden Ausgangsverbindungen hergestellt werden.

| **Beispiel** | **Struktur** | **Analytische Daten** |
|---|---|---|
| **65** | 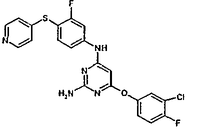 | LC-MS (Methode 13): Rₜ = 2.67 min MS (ESIpos): m/z = 458 (M+H)⁺ |
| **66** | 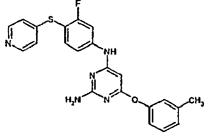 | LC-MS (Methode 13): Rₜ = 2.40 min MS (ESIpos): m/z = 420 (M+H)⁺ |
| **67** | 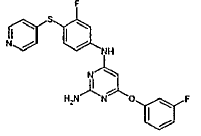 | LC-MS (Methode 13): Rₜ = 2.34 min MS (ESIpos): m/z = 424 (M+H)⁺ |
| **68** | 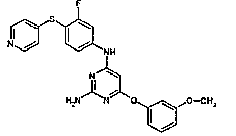 | LC-MS (Methode 13): Rₜ = 2.29 min MS (ESIpos): m/z = 436 (M+H)⁺ |
| **69** | 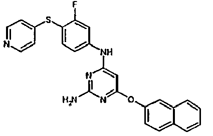 | LC-MS (Methode 15): Rₜ = 2.65 min MS (ESIpos): m/z = 456 (M+H)⁺ |
| **70** | 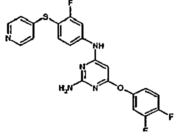 | LC-MS (Methode 13): Rₜ = 2.44 min MS (ESIpos): m/z = 442 (M+H)⁺ |
| **71** | 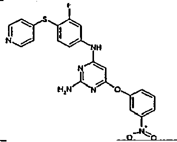 | LC-MS (Methode 13): Rₜ = 2.37 min MS (ESIpos): m/z = 451 (M+H)⁺ |
| **72** | 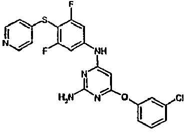 | LC-MS (Methode 5): Rₜ = 4.30 min MS (ESIpos): m/z = 458 (M+H)⁺ |
| **73** | 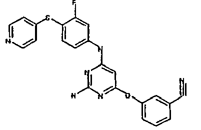 | LC-MS (Methode 5): Rₜ = 3.10 min MS (ESIpos): m/z = 431 (M+H)⁺ |
| **74** | 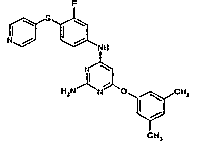 | LC-MS (Methode 5): Rₜ = 3.30 min MS (ESIpos): m/z = 434 (M+H)⁺ |
| **75** | 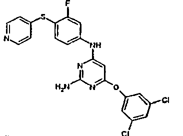 | LC-MS (Methode 13): Rₜ = 3.48 min MS (ESIpos): m/z = 474 (M+H)⁺ |
| **76** | 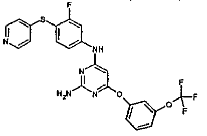 | LC-MS (Methode 13): Rₜ = 3.38 min MS (ESIpos): m/z = 490 (M+H)⁺ |
| **77** | 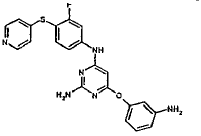 | LC-MS (Methode 13): Rₜ = 2.70 min MS (ESIpos): m/z = 421 (M+H)⁺ |
| **78** | 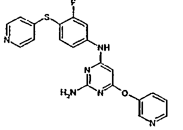 | LC-MS (Methode 13): Rₜ = 2.63 min MS (ESIpos): m/z = 407 (M+H)⁺ |
| **79** | 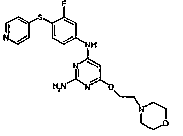 | LC-MS (Methode 13): Rₜ = 2.02 min MS (ESIpos): m/z = 443 (M+H)⁺ |
| **80** | 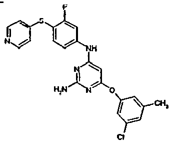 | LC-MS (Methode 14): Rₜ = 3.49 min MS (ESIpos): m/z = 454 (M+H)⁺ |
| **81** | 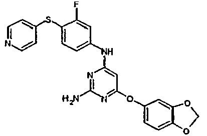 | LC-MS (Methode 13): Rₜ = 2.19 min MS (ESIpos): m/z = 450 (M+H)⁺ |

### Beispiel 82

### 1-(2-Amino-6-{[3-flluor-4-(4-pyridinylsulfanyl)phenyl]amino}-4-pyrimidinyl)-octahydropyrrolo[3,4-b]pyrrol-5-ium Trifluoracetat

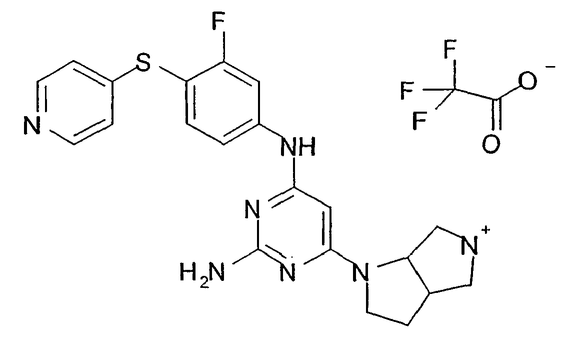

35 mg (0.067 mmol) tert.-Butyl-1-(3-Amino-5-{[3-fluor-4-(4-pyridinylsulfanyl)-phenyl]amino}phenyl)hexahydropyrrolo[3,4-b]pyrrol-5(1H)-carboxylat (aus Beispiel 60) werden in 1.25 ml Dichlormethan/Trifluoressigsäure (1/1) 30 min gerührt. Die Reaktionslösung wird im Vakuum zur Trockene eingeengt.

Man erhält 35 mg (97 % d. Th.) des Produktes.

HPLC (Methode 1 ): Rₜ = 3.20 min
MS (ESIpos): m/z = 424 (M+H)⁺

### Beispiel 83

### 1-(2-Amino-6-{[3-fluor-4-(4-pyridinylsulfanyl)phenyl]amino}-4-pyrimidinyl)-octahydropyrrolo[3,4-b]pyrrol

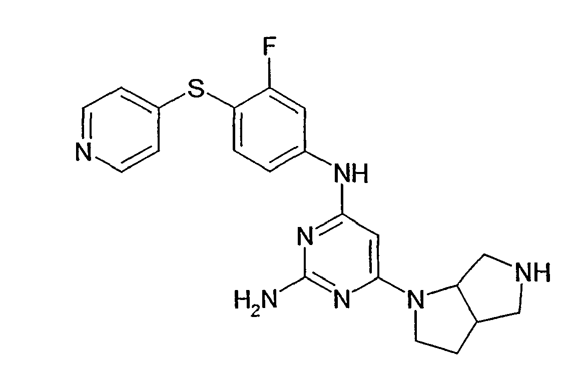

100 mg (0.86 mmol) 1-(2-Amino-6-{[3-fluor-4-(4-pyridinylsulfanyl)phenyl]amino}-4-pyrimidinyl)octahydropyrrolo[3,4-b]pyrrol-5-ium Trifluoracetat werden in 40 ml Dichlormethan/Methanol (20:1) gelöst und mit 40 ml 1N Natriumhydroxid-Lösung ausgeschüttelt, die organische Phase wird über Natriumsulfat getrocknet und im Vakuum einrotiert.

Man erhält 53 mg (67 % d. Th.) des Produktes.

LC-MS (Methodel3): Rₜ= 1.55 min
MS (ESIpos): m/z = 424 (M+H)⁺

### B. Bewertung der physiologischen Wirksamkeit

In einem in vitro-Assay mit rekombinanter Rho-Kinase-II wird die Hemmung des Enzyms untersucht. Die gefäßrelaxierende Wirkung wird an Phenylephrin-induzierten Kontraktionen isolierter Ringe der Kaninchen-Arteria-Saphena bestimmt. Die Eignung der erfindungsgemäßen Verbindungen zur Behandlung von kardiovaskulären Erkrankungen kann durch Untersuchung der blutdrucksenkenden Wirkung an narkotisierten Ratten gezeigt werden.

### Hemmung der rekombinanten Rho-Kinase II (ROKα)

Die Aktivität der Rho-Kinase wird durch den Einbau von ³³P-Phosphat in ein Substratpeptid bestimmt. Dazu wird kommerziell erhältliche Rho-Kinase II (Upstate Biotechnology) in Gegenwart des S6 Phosphate-Acceptor-Peptides mit den Testsubstanzen oder einer Lösungsmittelkontrolle 10 min bei 37°C vorinkubiert. Anschliessend wird die Kinase-Reaktion durch Zugabe von ³³P-markiertem ATP gestartet. Nach 20 min bei 37°C wird die Reaktion durch Zugabe von H₃PO₄ gestoppt. Aliquots werden auf Filter pipettiert, die Filter gewaschen und anschließend mit Scintillator beschichtet. Die Radioaktivität der am Filter gebundenen ³³P-markierten Peptide wird in einem Micro-Beta-Counter gemessen. Der IC₅₀-Wert entspricht der Konzentration einer Testsubstanz bei welcher der Rho-Kinase katalysierte Einbau von ³³P in das Peptid im Vergleich zu einer Lösungsmittelkontrolle um 50 % gehemmt ist. Die experimentellen Daten sind in der folgenden Tabelle zusammengestellt.

| **Beispiel-Nr.** | **IC₅₀ (nM)** |
|---|---|
| 1 | 7 |
| 20 | 11 |
| 22 | 6 |
| 56 | 70 |
| 59 | 5 |

### Gefäßrelatierende Wirkung in vitro

3 mm breite Ringe der isolierten Kaninchen-Arteria-Saphena werden einzeln in 5 ml-Organbäder mit 37°C warmer, carbogenbegaster Krebs-Henseleit-Lösung gebracht. Der Gefäßtonus wird isometrisch erfasst und registriert. Kontraktionen werden durch Zugabe von 3x10⁻⁸ g/ml Phenylephrin induziert und nach 4 min wieder ausgewaschen. Nach mehreren Kontrollzyklen wird die zu untersuchende Substanz mit jedem weiteren Durchgang in steigender Dosierung vorinkubiert und die darauffolgende Kontraktion mit der Höhe der letzten Kontrollkontraktion verglichen. Es wird die Konzentration errechnet, die erforderlich ist, um die Höhe des Kontrollwertes um 50 % zu reduzieren (IC₅₀). Die experimentellen Daten sind in der folgenden Tabelle zusammengestellt.

| **Beispiel-Nr.** | **IC₅₀ (nM)** |
|---|---|
| 1 | 760 |
| 20 | 1700 |
| 22 | 900 |
| 59 | 360 |

### Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingerührt. Die zu prüfenden Substanzen werden als Lösungen entweder oral mittels Schlundsonde oder über die Femoralvene intravenös verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

## Patentansprüche

1. Verbindungen der Formel 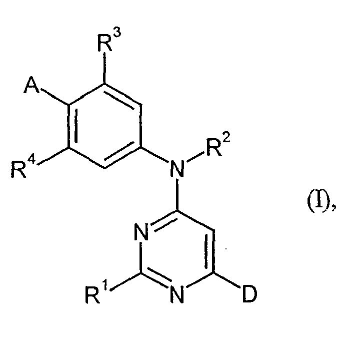 worin
R¹ Amino oder Hydroxy bedeutet,
R² Wasserstoff, (C₁-C₆)-Alk-yl oder (C₃-C₈)-Cycloalkyl bedeutet,
R³ und R⁴ unabhängig voneinander Cyano, Wasserstoff, Fluor oder Chlor bedeuten,
A einen Rest 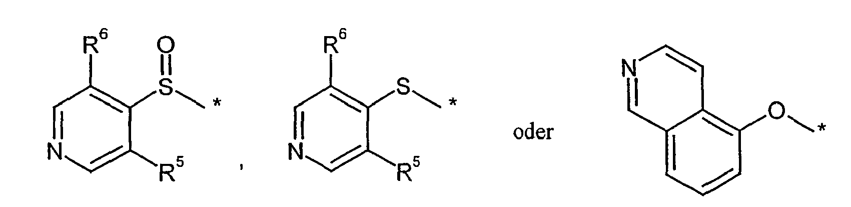 bedeutet, worin
R⁵ und R⁶ unabhängig voneinander Wasserstoff, Fluor oder Chlor bedeuten,
D
(1) einen Rest bedeutet, der ausgewählt ist aus der Gruppe von
Phenyl, das seinerseits durch (C₁-C₄)-Alkylcarbonylamino, Hydroxymethyl, Cyano, (C₁-C₄)-Alkoxymethyl oder 1,2-Dioxymethylen substituiert ist,
Chinolin, Isochinolin, Indol oder 6-gliedriges Heteroaryl mit 2 oder 3 Stickstoffatomen, wobei die Ringe jeweils über ein Kohlenstoffatom verknüpft sind,
Pyridylmethyl, 2-Oxo-2H-pyridin-1-yl, 4-Oxo-4H-pyridin-1-yl, die ihrerseits durch Fluor, Chlor oder (C₁-C₄)-Alkyl substituiert sein können, und
Pyridyl, das seinerseits durch Fluor, Chlor oder (C₁-C₄)-Alkyl substituiert ist,
oder
(2) einen Rest *-OR⁷ bedeutet,
worin
R⁷ Phenyl, das durch (C₁-C₄)-Alkyl oder (C₁-C₄)-Alkoxy, die ihrerseits durch Hydroxy und/oder *-NR⁸R⁹ sub- stituiert sein können, Trifluormethyl, Trifluormethoxy, Nitro, Cyano, *-NR⁸R⁹; Fluor, Chlor oder 1,2-Dioxy- methylen substituiert sein kann,
3- bis 7-gliedriges Heterocyclyl mit einem Stickstoffatom, das durch Wasserstoff, (C₁-C₆)-Alkyl oder (C₃-C₈)-Cycloalkyl substituiert sein kann,
5- oder 6-gliedriges Heteroaryl mit bis zu drei Stickstoffatomen,
(C₁-C₆)-Alkyl oder (C₃-C₇)-Cycloallcyl, die ihrerseits durch Hydroxy oder *-NR⁸R⁹ substituiert sein können,
Thienyl, Furyl, Pyridylmethyl, Naphthyl oder Benzyl bedeutet,
worin
R⁸ und R⁹ unabhängig voneinander Wasserstoff oder (C₁-C₄)-Alkyl, das seinerseits durch Hydroxy oder Amino substituiert sein kann, bedeuten
oder
R⁸ und R⁹ gemeinsam mit dem Stickstoffatom, an das sie gebunden sind, einen 5- bis 7-gliedrigen Heterocyclus bilden, der noch ein Sauerstoffatom oder eine Gruppe N-H oder N-(C₁-C₄)-Alkyl im Ring aufweisen kann,
oder
(3) einen Rest *-NR¹⁰R¹¹ bedeutet,
worin
R¹⁰ Wasserstoff oder (C₁-C₄)-Alkyl bedeutet und
R¹¹ einen Rest *-(CH₂)ₓ-Phenyl, wobei Phenyl bis zu vierfach, unabhängig voneinander, durch Fluor, Chlor oder (C₁-C₄)-Alkyl substituiert sein kann, oder *-(CH₂)_{y}-E bedeutet,
worin
x 1, 2 oder 3 bedeutet,
y 0, 1, 2 oder 3 bedeutet und
E Pyrrolidin oder Piperidin, die ihrerseits durch (C₁-C₄)-Alkyl substituiert sein können, oder Pyridyl, das bis zu vierfach, unabhängig vonein- ander, durch Fluor, Chlor oder (C₁-C₄)-Alkyl substituiert sein kann, bedeutet,
oder
R¹⁰ und R¹¹ gemeinsam mit dem Stickstoffatom, an das sie gebunden sind, einen 5- oder 6-gliedrigen Heterocyclus bilden, der durch *-NR¹²R¹³, 1,1-Dioxyethylen, 5- oder 6-gliedriges Heterocyclyl mit einem oder zwei Heteroatomen N und/oder O, das seinerseits durch (C₁-C₄)-Alkyl substituiert sein kann, (C₁-C₄)-Alkoxy, durch Hydroxy oder (C₁-C₄)-Alkoxy substituiertes (C₁-C₄)-Alkyl, oder (C₁-C₄)-Alkoxycarbonyl substituiert ist, worin
R¹² und R¹³ unabhängig voneinander Wasserstoff, (C₁-C₆)-Alkyl, (C₁-C₄)-Alkoxycarbonyl, (C₃-C₈)-Cycloalkyl oder (C₁-C₄)-Alkanoyl bedeuten oder
R¹² und R¹³ gemeinsam mit dem Stickstoffatom, an das sie gebunden sind, einen 5- oder 6-gliedrigen Heterocyclus bilden,
oder
R¹⁰ und R¹¹ gemeinsam mit dem Stickstoffatom, an das sie
gebunden sind, einen 7- bis 12-gliedrigen bicyclischen Heterocyclus bilden, der anneliert oder spirocyclisch ist und ein oder zwei weitere Heteroatomen aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
oder
R¹⁰ und R¹¹ gemeinsam mit dem Stickstoffatom, an das sie gebunden sind, einen Rest 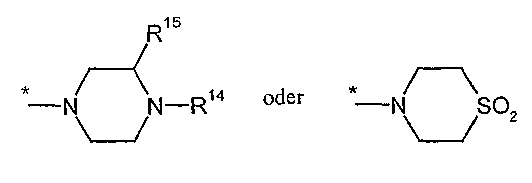 bilden, worin
R¹⁴ (C₂-C₆)-Alkenyl, (C₁-C₄)-Alkoxycarbonyl, oder *-(CH₂)_{z}-G bedeutet,
worin
z 0 oder 1 bedeutet und
G (C₃-C₈)-Cycloalkyl, Pyridyl, gegebenen- falls durch (C₁-C₄)-Alkyl oder (C₁-C₄)- Alkoxy substituiertes Phenyl, Tetra- hydrofuran oder 1,3-Dioxolan bedeutet,
und
R¹⁵ Wasserstoff oder (C₁-C₄)-Alkyl bedeutet,
und ihre Salze, Hydrate, Hydrate der Salze und Solvate.

2. Verbindungen der Formel (I) nach Anspruch 1, worin
R¹ Amino bedeutet,
R² Wasserstoff bedeutet,
R³ und R⁴ unabhängig voneinander Wasserstoff, Fluor oder Chlor bedeuten,
A einen Rest 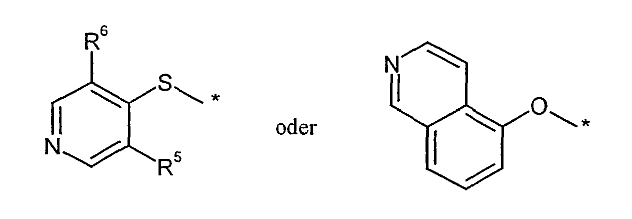
bedeutet, worin
R⁵ und R⁶ Wasserstoff bedeuten,
D
(1) einen Rest bedeutet, der ausgewählt ist aus der Gruppe von
Phenyl, das durch (C₁-C₄)-Alkylcarbonylamino, Hydroxymethyl, (C₁-C₄)-Alkoxymethyl oder 1,2-Dioxymethylen substituiert ist,
Chinolin, Indol oder 6-gliedriges Heteroaryl mit 2 oder 3 Stickstoffatomen, wobei die Ringe jeweils über ein Kohlenstoffatom verknüpft sind,
Pyridylmethyl, das durch (C₁-C₄)-Alkyl substituiert sein kann,
und
Pyridyl, das durch (C₁-C₄)-Alkyl substituiert ist,
oder
(2) einen Rest *-OR⁷ bedeutet,
worin
R⁷ Phenyl, das durch Fluor, Chlor, (C₁-C₄)-Alkyl, (C₁-C₄)-Alkoxy oder 1,2-Dioxymethylen substituiert sein kann,
(C₁-C₆)-Alkyl oder (C₃-C₈)-Cycloalkyl, die ihrerseits durch Hydroxy oder *-NR⁸R⁹ substituiert sein können,
Naphthyl oder Benzyl bedeutet,
worin
R⁸ und R⁹ unabhängig voneinander Wasserstoff oder (C₁-C₄)-Alkyl bedeuten
oder
R⁸ und R⁹ gemeinsam mit dem Stickstoffatom, an das sie gebunden sind, einen 5- bis 7-gliedrigen Heterocyclus bilden, der noch ein Sauerstoffatom oder eine Gruppe N-H oder N-(C₁-C₄)-Alkyl im Ring aufweisen kann,
oder
(3) einen Rest *-NR¹⁰R¹¹ bedeutet,
worin
R¹⁰ Wasserstoff oder (C₁-C₄)-Alkyl bedeutet und
R¹¹ einen Rest *-(CH₂)ₓ-Phenyl, wobei Phenyl bis zu vierfach, unabhängig voneinander, durch Fluor, Chlor oder (C₁-C₄)-Alkyl substituiert sein kann, oder *-(CH₂)_{y}-E bedeutet,
worin
x 1 oder 2 bedeutet,
y 0, 1 oder 2 bedeutet und
E Pyrrolidin oder Piperidin, die ihrerseits durch (C₁-C₄)-Alkyl substituiert sein können, oder Pyridyl, das bis zu vierfach, unabhängig von- einander, durch Fluor, Chlor oder (C₁-C₄)-Alkyl substituiert sein kann, bedeutet,
oder
R¹⁰ und R¹¹ gemeinsam mit dem Stickstoffatom, an das sie gebunden sind, einen 5- oder 6-gliedrigen Heterocyclus bilden, der durch *-NR¹²R¹³, 1,1-Dioxymethylen, 5-oder 6-gliedriges Heterocyclyl mit einem oder zwei Heteroatomen N und/oder O, das seinerseits durch (C₁-C₄)-Alkyl substituiert sein kann, oder (C₁-C₄)-Alkoxymethyl substituiert ist,
worin
R¹² und R¹³ unabhängig voneinander Wasserstoff, (C₁-C₆)-Allyl, (C₃-C₈)-Cycloalkyl oder (C₁-C₄)-Alkanoyl bedeuten
oder
R¹² und R¹³ gemeinsam mit dem Stickstoffatom, an das sie gebunden sind, einen 5- oder 6-gliedrigen Heterocyclus bilden,
oder
R¹⁰ und R¹¹ gemeinsam mit dem Stickstoffatom, an das sie gebunden sind, einen 8- bis 10-gliedrigen bicyclischen Heterocyclus bilden, der anneliert oder spirocyclisch ist und ein oder zwei weitere Heteroatomen aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
oder
R¹⁰ und R¹¹ gemeinsam mit dem Stickstoffatom, an das sie gebunden sind, einen Rest 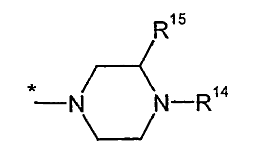 bilden,
worin
R¹⁴ (C₃-C₈)-Cycloalkyl, (C₂-C₆)-Alkenyl, (C₁-C₄)- Alkoxycarbonyl oder Tetrahydrofuran-2-yl- methyl bedeutet
und
R¹⁵ Wasserstoff oder (C₁-C₄)-Alkyl bedeutet,
und ihre Salze, Hydrate, Hydrate der Salze und Solvate.

3. Verbindungen der Formel (I) nach Anspruch 1
worin
R¹ Amino bedeutet,
R² Wasserstoff bedeutet,
R³ und R⁴ unabhängig voneinander Wasserstoff, Fluor oder Chlor bedeuten,
A einen Rest 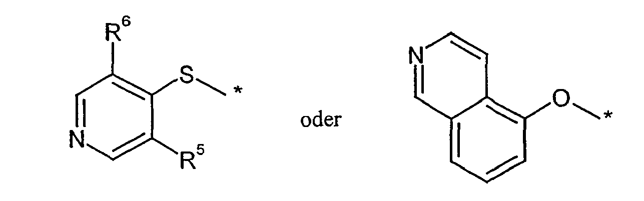
bedeutet, worin
R⁵ und R⁶ Wasserstoff bedeuten,
D
(1) einen Rest bedeutet, der ausgewählt ist aus der Gruppe von
Chinolin, Indol, Pyrazin, Pyridazin und Triazin, wobei die Ringe jeweils über ein Kohlenstoffatom verknüpft sind,
oder
(2) einen Rest *-OR⁷ bedeutet,
worin
R⁷ Phenyl, das durch Fluor, Chlor, (C₁-C₄)-Alkyl, (C₁-C₄)-Alkoxy oder 1,2-Dioxymethylen substituiert sein kann,
(C₁-C₆)-Alkyl oder (C₃-C₈)-Cycloalkyl, die ihrerseits durch Hydroxy oder *-NR⁸R⁹ substituiert sein können, bedeutet,
worin
R⁸ and R⁹ unabhängig voneinander Wasserstoff oder (C₁-C₄)-Alkyl bedeuten
oder
R⁸ und R⁹ gemeinsam mit dem Stickstoffatom, an das sie gebunden sind, einen Morpholin- oder Piperazinring bilden, wobei das zweite Stickstoffatom des Piperazinringes durch (C₁-C₄)-Alkyl substituiert sein kann,
oder
(3) einen Rest *-NR¹⁰R¹¹ bedeutet,
worin
R¹⁰ Wasserstoff oder (C₁-C₄)-Alhyl bedeutet und
R¹¹ einen Rest *-(CH₂)_{y}-E bedeutet,
worin
y 0 oder 1 bedeutet und
E Pyrrolidin oder Pyridyl, die ihrerseits durch (C₁-C₄)-Alkyl substituiert sein können, bedeutet,
oder
R¹⁰ und R¹¹ gemeinsam mit dem Stickstoffatom, an das sie gebunden sind, einen Pyrrolidin- oder Piperidinring bilden, die durch *-NR¹²R¹³, 1,1-Dioxymethylen, 5-oder 6-gliedriges Heterocyclyl mit einem oder zwei Heteroatomen N und/oder O, das seinerseits durch (C₁-C₄)-Alkyl substituiert sein kann, oder (C₁-C₄)-Alkoxymethyl substituiert ist,
worin
R¹² und R¹³ unabhängig voneinander Wasserstoff, (C₁-C₆)-Alkyl, (C₃-C₈)-Cycloalkyl oder (C₁-C₄)-Alkanoyl bedeuten oder
R¹² und R¹³ gemeinsam mit dem Stickstoffatom, an das sie gebunden sind, einen 5- oder 6-gliedrigen Heterocyclus bilden,
oder
R¹⁰ und R¹¹ gemeinsam mit dem Stickstoffatom, an das sie gebunden sind, einen 8- bis 10-gliedrigen bicyclischen Heterocyclus bilden, der anneliert oder spirocyclisch ist und ein oder zwei weitere Heteroatomen aus der Reihe N und/oder O im Ring aufweisen kann und der durch (C₁-C₄)-Alkyl, (C₁-C₄)-Alkoxycarbonyl, (C₁-C₄)-Alkanoyl oder Benzyl substituiert sein kann,
und ihre Salze, Hydrate, Hydrate der Salze und Solvate.

4. Verfahren zur Herstellung von Verbindungen der Formel (I) nach Anspruch 1, **dadurch gekennzeichnet, dass** man entweder
[A] Verbindungen der Formel (II) 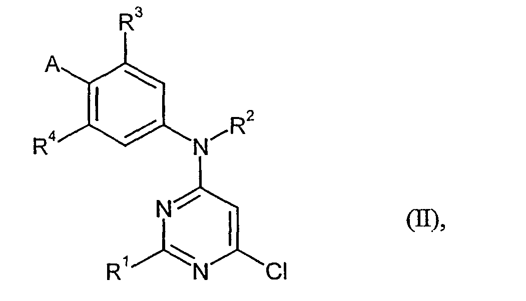 worin
A, R¹, R², R³ und R⁴ die in Anspruch 1 angegebene Bedeutung aufweisen,
mit Verbindungen der Formel (III)
D-X¹ (III)
worin
D die in Anspruch 1 angegebene Bedeutung aufweist und
X¹ für Wasserstoff oder *-B(OH)₂ steht,
oder
[B] Verbindungen der Formel (IV) 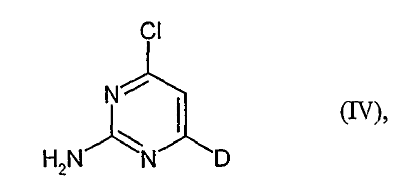 worin
D die in Anspruch 1 angegebene Bedeutung aufweist,
mit Verbindungen der Formel (V) 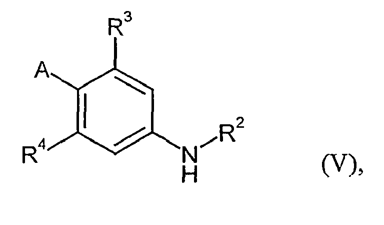 worin
A, R², R³ und R⁴ die in Anspruch 1 angegebene Bedeutung aufweisen,
umsetzt.

5. Verbindungen der Formel (I), wie in Anspruch 1 definiert, zur Behandlung und/oder Prophylaxe von Erkrankungen.

6. Arzneimittel enthaltend mindestens eine Verbindung der Formel (I), wie in Anspruch 1 definiert, und mindestens einen Hilfsstoff.

7. Arzneimittel enthaltend mindestens eine Verbindung der Formel (I), wie in Anspruch 1 definiert, und mindestens einen weiteren Wirkstoff.

8. Verwendung von Verbindungen der Formel (I), wie in Anspruch 1 definiert, zur Herstellung von Arzneimitteln zur Behandlung und/oder Prophylaxe von kardiovaskulären Erkrankungen.

## Claims

1. Compounds of the formula 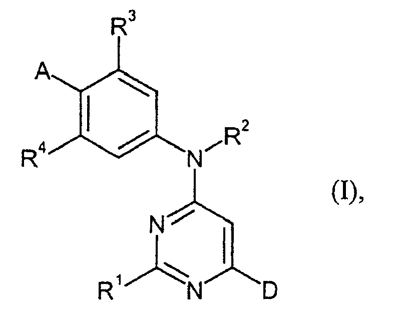 in which
R¹ represents amino or hydroxyl,
R² represents hydrogen, (C₁-C₆)-alkyl or (C₃-C₈)-cycloalkyl,
R³ and R⁴ independently of one another represent cyano, hydrogen, fluorine or chlorine,
A represents a radical 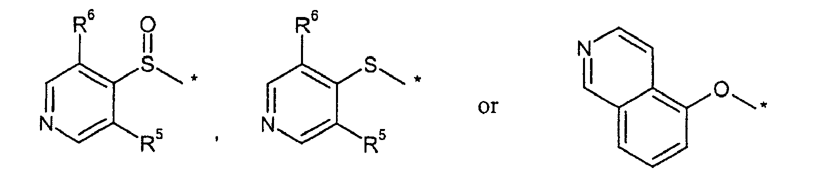
in which
R⁵ and R⁶ independently of one another represent hydrogen, fluorine or chlorine,
D
(1) represents a radical selected from the group consisting of
phenyl, which for its part is substituted by (C₁-C₄)-alkylcarbonylamino, hydroxymethyl, cyano, (C₁-C₄)-alkoxymethyl or 1,2-dioxymethylene,
quinoline, isoquinoline, indole or 6-membered heteroaryl having 2 or 3 nitrogen atoms, where the rings are in each case attached via a carbon atom,
pyridylmethyl, 2-oxo-2H-pyridin-1-yl, 4-oxo-4H-pyridin-1-yl, which for their part may be substituted by fluorine, chlorine or (C₁-C₄)-alkyl, and
pyridyl, which for its part is substituted by fluorine, chlorine or (C₁-C₄)-alkyl,
or
(2) represents a radical *-OR⁷,
in which
R⁷ represents phenyl which may be substituted by trifluoromethyl, trifluoromethoxy, nitro, cyano, *-NR⁸R⁹, fluorine, chlorine or 1,2-dioxymethylene or by (C₁-C₄)-alkyl or (C₁-C₄)-alkoxy, which for their part may be substituted by hydroxyl and/or *- NR⁸R⁹,
3- to 7-membered heterocyclyl having a nitrogen atom which may be substituted by hydrogen, (C₁-C₆)-alkyl or (C₃-C₈)-cycloalkyl,
5- or 6-membered heteroaryl having up to three nitrogen atoms,
(C₁-C₆)-alkyl or (C₃-C₇)-cycloalkyl which for their part may be substituted by hydroxyl or *- NR⁸R⁹,
thienyl, furyl, pyridylmethyl, naphthyl or benzyl,
in which
R⁸ and R⁹ independently of one another represent hydrogen or (C₁-C₄)-alkyl which for its part may be substituted by hydroxyl or amino,
or
R⁸ and R⁹ together with the nitrogen atom to which they are attached form a 5- to 7-membered heterocycle which may have an additional oxygen atom or a group N-H or N-(C₁-C₄)-alkyl in the ring,
or
(3) represents a radical *-NR¹⁰R¹¹,
in which
R¹⁰ represents hydrogen or (C₁-C₄)-alkyl and
R¹¹ represents a radical *-(CH₂)ₓ-phenyl, where phenyl may be substituted up to four times independently of one another by fluorine, chlorine or (C₁-C₄)-alkyl, or represents *-(CH₂)_{y}-E,
in which
x represents 1, 2 or 3,
y represents 0, 1, 2 or 3 and
E represents pyrrolidine or piperidine, which for their part may be substituted by (C₁-C₄)-alkyl, or represents pyridyl which may be substituted up to four times independently of one another by fluorine, chlorine or (C₁-C₄)-alkyl,
or
R¹⁰ and R¹¹ together with the nitrogen atom to which they are attached form a 5- or 6-membered heterocycle which is substituted by *-NR¹²R¹³, 1,1-dioxyethylene, (C₁-C₄)-alkoxy, hydroxyl- or (C₁-C₄)-alkoxy-substituted (C₁-C₄)-alkyl, (C₁-C₄)-alkoxycarbonyl or 5- or 6-membered heterocyclyl having one or two heteroatoms N and/or O, which for its part may be substituted by (C₁-C₄)-alkyl,
in which
R¹² and R¹³ independently of one another represent hydrogen, (C₁-C₆)-alkyl, (C₁-C₄)-alkoxycarbonyl, (C₃-C₈)-cycloalkyl or (C₁-C₄)-alkanoyl or
R¹² and R¹³ together with the nitrogen atom to which they are attached form a 5- or 6-membered heterocycle,
or
R¹⁰ and R¹¹ together with the nitrogen atom to which they are attached form a 7- to 12-membered bicyclic heterocycle which is fused or spirocyclic and may have one or two further heteroatoms from the group consisting of N and O in the ring and which may be substituted by (C₁-C₄)-alkyl, (C₁-C₄)-alkoxycarbonyl, (C₁-C₄)-alkanoyl or benzyl,
or
R¹⁰ and R¹¹ together with the nitrogen atom to which they are attached form a radical 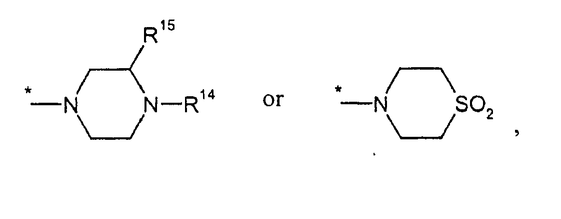 in which
R¹⁴ represents (C₂-C₆)-alkenyl, (C₁-C₄)- alkoxycarbonyl or *-(CH₂)_{z}-G,
in which
z represents 0 or 1 and
G represents (C₃-C₈)-cycloalkyl, pyridyl, optionally (C₁-C₄)-alkyl- or (C₁-C₄)-alkoxy- substituted phenyl, tetrahydrofuran or 1,3- dioxolane,
and
R¹⁵ represents hydrogen or (C₁-C₄)-alkyl,
and their salts, hydrates, hydrates of the salts and solvates.

2. Compounds of the formula (I) according to Claim 1, in which
R¹ represents amino,
R² represents hydrogen,
R³ and R⁴ independently of one another represent hydrogen, fluorine or chlorine,
A represents a radical 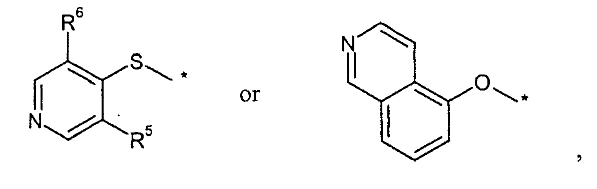
in which
R⁵ and R⁶ represent hydrogen,
D
(1) represents a radical selected from the group consisting of
phenyl which is substituted by (C₁-C₄)-alkylcarbonylamino, hydroxymethyl, (C₁-C₄)-alkoxymethyl or 1,2-dioxymethylene,
quinoline, indole or 6-membered heteroaryl having 2 or 3 nitrogen atoms, where the rings are in each case attached via a carbon atom,
pyridylmethyl, which may be substituted by (C₁-C₄)-alkyl,
and
pyridyl, which is substituted by (C₁-C₄)-alkyl,
or
(2) represents a radical *-OR⁷,
in which
R⁷ represents phenyl, which may be substituted by fluorine, chlorine, (C₁-C₄)-alkyl, (C₁-C₄)-alkoxy or 1,2-dioxymethylene,
(C₁-C₆)-alkyl or (C₃-C₈)-cycloalkyl, which for their part may be substituted by hydroxyl or *-NR⁸R⁹,
naphthyl or benzyl,
in which
R⁸ and R⁹ independently of one another represent hydrogen or (C₁-C₄)-alkyl
or
R⁸ and R⁹ together with the nitrogen atom to which they are attached form a 5- to 7-membered heterocycle which may have an additional oxygen atom or a group N-H or N-(C₁-C₄)-alkyl in the ring,
or
(3) represents a radical *-NR¹⁰R¹¹,
in which
R¹⁰ represents hydrogen or (C₁-C₄)-alkyl and
R¹¹ represents a radical *-(CH₂)ₓ-phenyl, where phenyl may be substituted up to four times independently of one another by fluorine, chlorine or (C₁-C₄)-alkyl, or represents *-(CH₂)_{y}-E,
in which
x represents 1 or 2,
y represents 0, 1 or 2 and
E represents pyrrolidine or piperidine, which for their part may be substituted by (C₁-C₄)-alkyl, or represents pyridyl which may be substituted up to four times independently of one another by fluorine, chlorine or (C₁-C₄)-alkyl,
or
R¹⁰ and R¹¹ together with the nitrogen atom to which they are attached form a 5- or 6-membered heterocycle which is substituted by *-NR¹²R¹³, 1,1-dioxymethylene, (C₁-C₄)-alkoxymethyl or by 5- or 6-membered heterocyclyl having one or two heteroatoms N and/or O, which for its part may be substituted by (C₁-C₄)-alkyl,
in which
R¹² and R¹³ independently of one another represent hydrogen, (C₁-C₆)alkyl, (C₃-C₈)-cycloalkyl or (C₁-C₄)-alkanoyl
or
R¹² and R¹³ together with the nitrogen atom to which they are attached form a 5- or 6-membered heterocycle,
or
R¹⁰ and R¹¹ together with the nitrogen atom to which they are attached form an 8- to 10-membered bicyclic heterocycle which is fused or spirocyclic and may have one or two further heteroatoms from the group consisting of N and O in the ring and which may be substituted by (C₁-C₄)-alkyl, (C₁-C₄)-alkoxycarbonyl, (C₁-C₄)-alkanoyl or benzyl,
or
R¹⁰ and R¹¹ together with the nitrogen atom to which they are attached form a radical 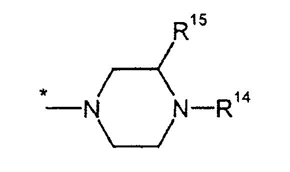 in which
R¹⁴ represents (C₃-C₈)-cycloalkyl, (C₂-C₆)-alkenyl, (C₁-C₄)-alkoxycarbonyl or tetrahydrofuran-2- ylmethyl,
and
R¹⁵ represents hydrogen or (C₁-C₄)-alkyl,
and their salts, hydrates, hydrates of the salts and solvates.

3. Compounds of the formula (I) according to Claim 1, in which
R' represents amino,
R² represents hydrogen,
R³ and R⁴ independently of one another represent hydrogen, fluorine or chlorine,
A represents a radical 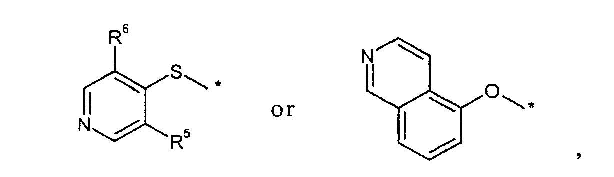
in which
R⁵ and R⁶ represent hydrogen,
D
(1) represents a radical which is selected from the group consisting of
quinoline, indole, pyrazine, pyridazine and triazine, where the rings are in each case attached via a carbon atom,
or
(2) represents a radical *-OR⁷
in which
R⁷ represents phenyl which may be substituted by fluorine, chlorine, (C₁-C₄)-alkyl, (C₁-C₄)-alkoxy or 1,2-dioxymethylene,
(C₁-C₆)-alkyl or (C₃-C₈)-cycloalkyl which for their part may be substituted by hydroxyl or *-NR⁸R⁹,
in which
R⁸ and R⁹ independently of one another represent hydrogen or (C₁-C₄)-alkyl
or
R⁸ and R⁹ together with the nitrogen atom to which they are attached form a morpholine or piperazine ring, where the second nitrogen atom of the piperazine ring may be substituted by (C₁-C₄)-alkyl,
or
(3) represents a radical *-NR¹⁰R¹¹,
in which
R¹⁰ represents hydrogen or (C₁-C₄)-alkyl and
R¹¹ represents a radical *-(CH_{z})_{y}-E,
in which
y represents 0 or 1 and
E represents pyrrolidine or pyridyl, which for their part may be substituted by (C₁-C₄)-alkyl,
or
R¹⁰ and R¹¹ together with the nitrogen atom to which they are attached form a pyrrolidine or piperidine ring which may be substituted by *-NR¹²R¹³, 1,1-dioxymethylene, (C₁-C₄)-alkoxymethyl or 5- or 6-membered heterocyclyl having one or two heteroatoms N and/or O, which for its part may be substituted by (C₁-C₄)-alkyl,
in which
R¹² and R¹³ independently of one another represent hydrogen, (C₁-C₆)-alkyl, (C₃-C₈)-cycloalkyl or (C₁-C₄)-alkanoyl or
R¹² and R¹³ together with the nitrogen atom to which they are attached form a 5- or 6-membered heterocycle,
or
R¹⁰ and R¹¹ together with the nitrogen atom to which they are attached form an 8- to 10-membered bicyclic heterocycle which is fused or spirocyclic and may have one or two further heteroatoms from the group consisting of N and O in the ring and which may be substituted by (C₁-C₄)-alkyl, (C₁-C₄)-alkoxycarbonyl, (C₁-C₄)-alkanoyl or benzyl,
and their salts, hydrates, hydrates of the salts and solvates.

4. Process for preparing compounds of the formula (I) according to Claim 1, **characterized in that** either
[A] compounds of the formula (II) 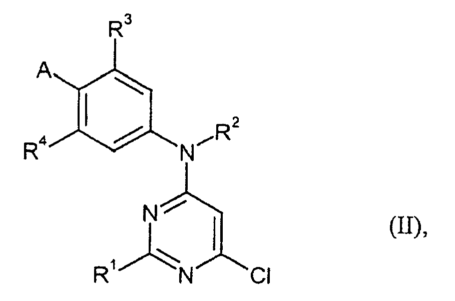 in which
A, R¹, R², R³ and R⁴ are as defined in Claim 1,
are reacted with compounds of the formula (III)
D-X¹ (III)
in which
D is as defined in Claim 1 and
X¹ represents hydrogen or *-B(OH)₂
or
[B] compounds of the formula (IV) 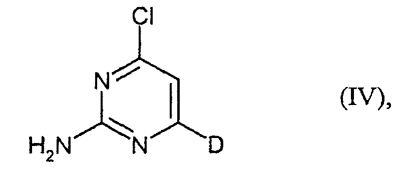 in which
D is as defined in Claim 1
are reacted with compounds of the formula (V) 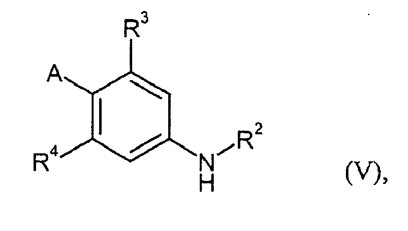 in which
A, R², R³ and R⁴ are as defined in Claim 1.

5. Compounds of the formula (I) as defined in Claim 1 for the treatment and/or prophylaxis of disorders.

6. Pharmaceutical, comprising at least one compound of the formula (I) as defined in Claim 1 and at least one auxiliary.

7. Pharmaceutical, comprising at least one compound of the formula (I) as defined in Claim 1 and at least one further active compound.

8. Use of compounds of the formula (I) as defined in Claim 1 for preparing pharmaceuticals for the treatment and/or prophylaxis of cardiovascular disorders.

## Revendications

1. Composés de formule 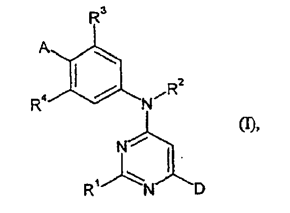 dans laquelle
R¹ est un groupe amino ou hydroxy,
R² est un atome d'hydrogène ou un groupe alkyle en C₁-C₆ ou cycloalkyle en C₃-C₈,
R³ et R⁴ représentent chacun indépendamment de l'autre un groupe cyano, un atome d'hydrogène, de fluor ou de chlore,
A est un radical 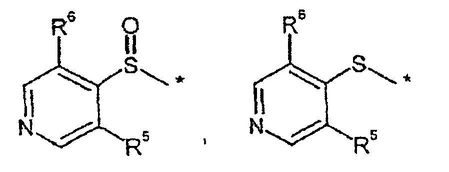 ou 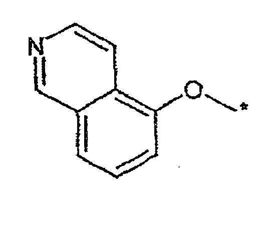 où
R⁵ et R⁶ représentent chacun indépendamment de l'autre l'atome d'hydrogène, de fluor ou de chlore,
D représente
(1) un radical choisi dans le groupe comprenant les groupes
phényle, qui pour sa part est substitué par un ou des substituants (alkyle en C₁-C₄)carbonylamino, hydroxyméthyle, cyano, (alcoxy en C₁-C₄)méthyle ou 1,2-dioxyméthylène,
quinoléine, isoquinoléine, indole, ou hétéroaryle à 6 chaînons ayant 2 ou 3 atomes d'azote, chacun des noyaux étant relié par l'intermédiaire d'un atome de carbone,
pyridylméthyle, 2-oxo-2H-pyridin-1-yle, 4-oxo-4H-pyridin-1-yle, qui pour sa part peuvent être substitués par un ou des substituants fluoro, chloro ou alkyle en C₁-C₄, et
pyridyle, qui pour sa part est substitué par un ou des substituants fluoro, chloro ou alkyle en C₁-C₄,
ou
(2) un radical *-OR⁷, où
R⁷ est un groupe phényle, qui peut être substitué par un ou des substituants alkyle en C₁-C₄ ou alcoxy en C₁-C₄, qui pour leur part peuvent être substitués par un ou des substituants hydroxy ou *-NR⁸R⁹ ; trifluorométhyle, trifluorométhoxy, nitro, cyano, *-NR⁸R⁹, fluoro, chloro ou 1,2-dioxyméthylène,
hétérocyclyle à 3 à 7 chaînons comportant un atome d'azote, qui peut être substitué par un ou des atomes d'hydrogène, groupes alkyle en C₁-C₆ ou cycloalkyle en C₃-C₈,
hétéroaryle à 5 ou 6 chaînons ayant jusqu'à trois atomes d'azote,
alkyle en C₁-C₆ ou cycloalkyle en C₃-C₇, qui pour leur part peuvent être substitués par un ou des substituants hydroxy ou *-NR⁸R⁹,
thiényle, furyle, pyridylméthyle, naphtyle ou benzyle,
où
R⁸ et R⁹ représentent chacun indépendamment de l'autre un atome d'hydrogène ou un groupe alkyle en C₁-C₄, qui pour sa part peut être substitué par un ou des substituants hydroxy ou amino, ou R⁸ et
R⁹, avec l'atome d'azote auquel ils sont liés, forment un radical hétérocyclique à 5 à 7 chaînons, qui peut encore comporter un atome d'oxygène ou un groupe N-H ou N-(alkyle en C₁-C₄) sur le noyau,
ou
(3) un radical *-NR¹⁰R¹¹,
dans lequel
R¹⁰ est un atome d'hydrogène ou un groupe alkyle en C₁-C₄ et
R¹¹ est un radical *-(CH_{z})ₓ-phényle, dans lequel le groupe phényle peut être substitué jusqu'à quatre fois, indépendamment les uns des autres, par un ou des substituants fluoro, chloro ou alkyle en C₁-C₄ ; ou *-(CH₂)_{y}-E, où x vaut 1, 2 ou 3, y vaut 0, 1, 2 ou 3, et E est la pyrrolidine ou la pipéridine, qui pour leur part peuvent être substitués par un ou des substituants alkyle en C₁-C₄, ou pyridyle, qui peut être substitué jusqu'à quatre fois, indépendamment les uns des autres, par un ou des substituants fluoro, chloro ou alkyle en C₁-C₄,
ou
R¹⁰ et R¹¹, avec l'atome d'azote auquel ils sont liés, forment un radical hétérocyclique à 5 ou 6 chaînons, qui est substitué par un ou des substituants *-NR¹²R¹³, 1,1-dioxyéthylène,
hétérocyclyle à 5 ou 6 chaînons ayant un ou deux hétéroatomes N et/ou O, qui pour sa part peuvent être substitués par un ou des substituants alkyle en C₁-C₄ ; alcoxy en C₁-C₄, alkyle en C₁-C₄ substitué par un ou des substituants hydroxy ou alcoxy en C₁-C₄ ; ou (alcoxy en C₁-C₄)carbonyle,
où
R¹² et R¹³ représentent chacun indépendamment de l'autre un atome d'hydrogène, un groupe alkyle en C₁-C₆, (alcoxy en C₁-C₄)carbonyle, cycloalkyle en C₃-C₈ ou alcanoyle en C₁-C₄, ou R¹² et R¹³, avec l'atome d'azote auquel ils sont liés, forment un radical hétérocyclique à 5 ou 6 chaînons,
ou
R¹⁰ et R¹¹, avec l'atome d'azote auquel ils sont liés, forment un radical hétérocyclique bicyclique à 7 à 12 chaînons, qui est condensé ou spirocyclique, et peut comporter dans le noyau un ou deux hétéroatomes choisis dans la série N et/ou O, et qui peut être substitué par un ou des substituants alkyle en C₁-C₄, (alcoxy en C₁-C₄)carbonyle, alcanoyle en C₁-C₄ ou benzyle, ou R¹⁰ et R¹¹, avec l'atome d'azote auquel ils sont liés, forment un radical 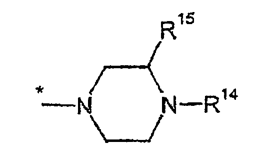 ou 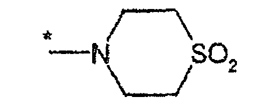 où
R¹⁴ est un groupe alcényle en C₂-C₆, (alcoxy en C₁-C₄)carbonyle ou *-(CH₂)_{z}-G, où z vaut 0 ou 1, et G est un groupe cycloalkyle en C₃-C₈, pyridyle, phényle éventuellement substitué par un ou des substituants alkyle en C₁-C₄ ou alcoxy en C₁-C₄, tétrahydrofuranne ou 1,3-dioxolanne,
et
R¹⁵ est un atome d'hydrogène ou un groupe alkyle en C₁-C₄,
et leurs sels, hydrates, hydrates des sels et produits de solvatation.

2. Composés de formule (I) selon la revendication 1, dans laquelle
R¹ est un groupe amino,
R² est un atome d'hydrogène,
R³ et R⁴ représentent chacun indépendamment de l'autre un atome d'hydrogène, de fluor ou de chlore,
A est un radical 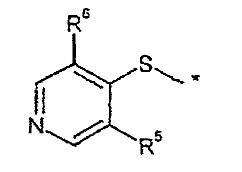 ou 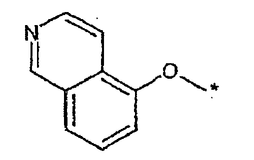 où
R⁵ et R⁶ sont des atomes d'hydrogène,
D représente
(1) un radical qui est choisi dans le groupe comprenant les groupes
phényle, qui est substitué par un ou des substituants (alkyle en C₁-C₄)carbonylamino, hydroxyméthyle, (alcoxy en C₁-C₄)méthyle ou 1,2-dioxyméthylène,
quinoléine, indole ou hétéroaryle à 6 chaînons, ayant 2 ou 3 atomes d'azote, chacun des noyaux étant lié par l'intermédiaire d'un atome de carbone,
pyridylméthyle, qui peut être substitué par un ou des substituants alkyle en C₁-C₄,
et
pyridyle, qui est substitué par ou des substituants alkyle en C₁-C₄,
ou
(2) un radical *-OR⁷,
où
R⁷ est un groupe phényle, qui peut être substitué par un ou plusieurs substituants fluoro, chloro, alkyle en C₁-C₄, alcoxy en C₁-C₄ ou 1,2-dioxyméthylène,
alkyle en C₁-C₆ ou cycloalkyle en C₃-C₈, qui pour leur part peuvent être substitués par un ou des substituants hydroxy *-NR⁸R⁹,
naphtyle ou benzyle,
où
R⁸ et R⁹ représentent chacun indépendamment de l'autre un atome d'hydrogène ou un groupe alkyle en C₁-C₄, ou R⁸ et R⁹, avec l'atome d'azote auquel ils sont liés, forment un radical hétérocyclique à 5 à 7 chaînons, qui peut comporter encore sur le noyau un atome d'oxygène ou un groupe N-H ou N-(alkyle en C₁-C₄),
ou
(3) un radical *-NR¹⁰R¹¹,
où
R¹⁰ est un atome d'hydrogène ou un groupe alkyle en C₁-C₄, et
R¹¹ est un groupe *-(CH₂)ₓ-phényle, où le groupe phényle peut être substitué jusqu'à quatre fois,
indépendamment les uns des autres, par un ou des substituants fluoro, chloro ou alkyle en C₁-C₄ ; ou *-(CH₂)_{y}-E, où x vaut 1 ou 2, y vaut 0, 1 ou 2, et E est la pyrrolidine ou la pipéridine, qui pour leur part peuvent être substituées par un ou des substituants alkyle en C₁-C₄, ou pyridyle, qui peut être substitué jusqu'à quatre fois, indépendamment les uns des autres, par un ou des substituants fluoro, chloro ou alkyle en C₁-C₄,
ou
R¹⁰ et R¹¹, avec l'atome d'azote auquel ils sont liés, forment un radical hétérocyclique à cinq ou six chaînons, qui est substitué par ou des substituants *-NR¹²R¹³, 1,1-dioxyméthylène, hétérocyclyle à 5 ou 6 chaînons ayant un ou deux hétéroatomes N et/ou O, qui pour leur part peuvent être substitués par un ou des substituants alkyle en C₁-C₄ ; ou (alcoxy en C₁-C₄)méthyle,
où
R¹² et R¹³ représentent chacun indépendamment de l'autre un atome d'hydrogène, un groupe alkyle en C₁-C₆, cycloalkyle en C₃-C₈ ou alcanoyle en C₁-C₄, ou
R¹² et R¹³, avec l'atome d'azote auquel ils sont liés, forment un radical hétérocyclique à 5 ou 6 chaînons,
ou
R¹⁰ et R¹¹, avec l'atome d'azote auquel ils sont liés, forment un radical hétérocyclique bicyclique à 8 à 10 chaînons, qui est condensé ou spirocyclique, et peut comporter sur le noyau un ou deux hétéroatomes supplémentaires choisis dans la série N et/ou O, et qui peut être substitué par un ou des substituants alkyle en C₁-C₄, par un (alcoxy en C₁-C₄)carbonyle, alcanoyle en C₁-C₄ ou benzyle, ou R¹⁰ et R¹¹, avec l'atome d'azote auquel ils sont liés, forment un radical 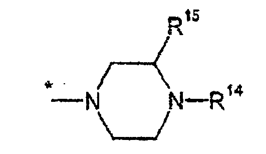 dans lequel
R¹⁴ est un groupe cycloalkyle en C₃-C₈, alcényle en C₂-C₆, (alcoxy en C₁-C₄)carbonyle ou tétrahydrofuranne-2-yl-méthyle,
et
R¹⁵ est un atome d'hydrogène ou un groupe alkyle en C₁-C₄,
et leurs sels, hydrates, hydrates des sels et produits de solvatation.

3. Composés de formule (I) selon la revendication 1, dans laquelle
R¹ est le groupe amino,
R² est un atome d'hydrogène,
R³ et R⁴ représentent chacun indépendamment de l'autre un atome d'hydrogène, de fluor ou de chlore,
A est un radical 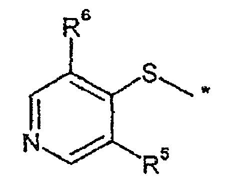 ou 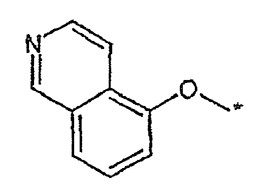 où
R⁵ et R⁶ sont des atomes d'hydrogène,
D représente
(1) un radical choisi dans le groupe comprenant les groupes
quinoléine, indole, pyrazine, pyridazine et triazine, chacun des noyaux étant lié par l'intermédiaire d'un atome de carbone, ou
(2) un radical *-OR⁷,
où
R⁷ est un groupe phényle, qui peut être substitué par un ou des substituants fluoro, chloro, alkyle en C₁-C₄, alcoxy en C₁-C₄ ou 1,2-dioxyméthylène, alkyle en C₁-C₆ ou cycloalkyle en C₃-C₆, qui pour leur part peuvent être substitués par un ou des substituants hydroxy ou *-NR⁸R⁹,
où
R⁸ et R⁹ représentent chacun indépendamment de l'autre un atome d'hydrogène ou un groupe alkyle en C₁-C₄, ou R⁸ et R⁹, avec l'atome d'azote auquel ils sont liés, forment un noyau morpholine ou pipérazine, le deuxième atome d'azote du noyau pipérazine pouvant être substitué par un substituant alkyle en C₁-C₄,
ou
(3) un radical *-NR¹⁰R¹¹,
où
R¹⁰ est un atome d'hydrogène ou un groupe alkyle en C₁-C₄, et R¹¹ est un radical *-(CH₂)_{y}-E, où y vaut 0 ou 1, et E est le groupe pyrrolidine ou pyridyle, qui pour leur part peuvent être substitués par un ou des substituants alkyle en C₁-C₄ ;
ou
R¹⁰ et R¹¹, avec l'atome d'azote auquel ils sont liés, forment un noyau pyrrolidine ou pipéridine, qui est substitué par un ou des substituants *-NR¹²R¹³, 1,1-dioxyméthylène, hétérocyclyle à 5 ou 6 chaînons ayant un ou deux hétéroatomes N et/ou O, qui pour leur part peuvent être substitués par un ou des substituants alkyle en C₁-C₄ ; ou (alcoxy en C₁-C₄)méthyle,
où
R¹² est R¹³ représentent chacun indépendamment de l'autre un atome d'hydrogène, un groupe alkyle en C₁-C₆, cycloalkyle en C₃-C₈ ou alcanoyle en C₁-C₄, ou R¹² et R¹³, avec l'atome d'azote auquel ils sont liés, forment un radical hétérocyclique à 5 ou 6 chaînons,
ou
R¹⁰ et R¹¹, avec l'atome d'azote auquel ils sont liés, forment un radical hétérocyclique bicyclique à 8 à 10 chaînons, qui est condensé ou spirocyclique, et qui peut comporter un ou deux autres hétéroatomes de la série N et/ou O, et qui peut être substitué par un ou des substituants alkyle en C₁-C₄, (alcoxy en C₁-C₄) carbonyle, alcanoyle en C₁-C₄ ou benzyle,
et leurs sels, hydrates, hydrates des sels et produits de solvatation.

4. Procédé de préparation de composés de formule (I) selon la revendication 1, **caractérisé en ce que**
(A) on fait réagir des composés de formule (II) 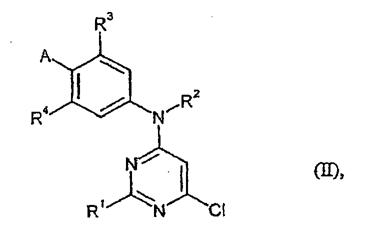 dans laquelle
A, R¹, R², R³ et R⁴ ont les significations données dans la revendication 1, avec des composés de formule (III)
D - X¹ (III)
dans laquelle
D a les significations données dans la revendication 1 et
X¹ est un atome d'hydrogène ou un radical *-B(OH)₂,
ou
(B) on fait réagir des composés de formule (IV) 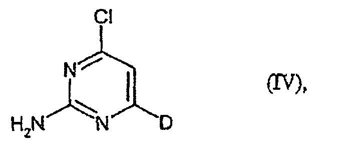 dans laquelle
D a les significations données dans la revendication 1,
avec des composés de formule (V) 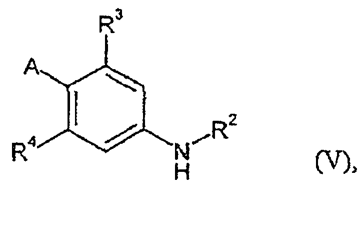 dans laquelle
A, R², R³ et R⁴ ont les significations données dans la revendication 1.

5. Composés de formule (I) tels que définis dans la revendication 1, pour le traitement et/ou la prophylaxie de maladies.

6. Médicament contenant au moins un composé de formule (I) tel que défini dans la revendication 1, et au moins un adjuvant.

7. Médicament contenant au moins un composé de formule (I) tel que défini dans la revendication 1, et au moins un principe actif supplémentaire.

8. Utilisation de composé de formule (I) tel que défini dans la revendication 1 pour préparer des médicaments destinés au traitement et/ou à la prophylaxie de maladies cardiovasculaires.
